# EUROPEAN PATENT SPECIFICATION

(11) **EP 0 935 003 B1**
(45) Date of publication and mention of the grant of the patent: **19.03.2003**
(21) Application number: 98310224.5
(22) Date of filing: 14.12.1998
(51) Int. Cl.: C12Q 1/68, C12N 15/90

(54) **Method for identification and detection of microorganisms using gyrase gene as an indicator**
Methode zur Identifizierung und dem Nachweis von Microorganismen mit Hilfe des Gyrase Gens
Méthode pour l'identification et la détection de micro-organismes au moyen du gène codant pour la gyrase

(30) Priority: 12.12.1997 JP 34331697
(43) Date of publication of application: 11.08.1999
(73) Proprietor: MARINE BIOTECHNOLOGY INSTITUTE CO., LTD., Bunkyo-ku, Tokyo 113-0033 (JP)
(72) Inventor: Yamamoto, Satoshi, Kamaishi Laboratory, Kamaishi-shi, Iwate 026-0001 (JP); Kasai, Hiroaki, Kamaishi Laboratory, Kamaishi-shi, Iwate 026-0001 (JP); Nakamura, Shoko, Kamaishi Laboratory, Kamaishi-shi, Iwate 026-0001 (JP); Suzuki, Makoto, Kamaishi Laboratory, Kamaishi-shi, Iwate 026-0001 (JP); Hamada, Tohru, Kamaishi Laboratory, Kamaishi-shi, Iwate 026-0001 (JP)
(74) Representative: Woods, Geoffrey Corlett

(56) References cited:
- WO-A-97/35970
- US-A- 5 645 994
- DATABASE WPI Week 9541 Derwent Publications Ltd., London, GB; AN 95-315932 XP002113794 "Identify detect microbe DNA gene sequence allow more accuracy define microbe strain species" & JP 07 213299 A (KAIYO BIOTECHNOLOGY KENKYUSHO KK), 15 August 1995 (1995-08-15)
- YAMAMOTO ET AL: 'PCR amplification and direct sequencing of gyrB genes with universal primers and their application to the detection and taxonomic analysis of Pseudomonas putida strains' APPLIED AND ENVIRONMENTAL MICROBIOLOGY vol. 61, no. 3, March 1995, pages 1104 - 1109

## Description

The present invention is involved in a method for the identification and detection of organisms using the sequences of their genes encoding the B subunit of the DNA gyrase.

This invention is useful in medical fields as well as various industrial fields where the identification/classification or detection/monitoring of specific microorganisms (bacteria, yeasts, fungi, archaea and bacteria), especially bacteria, is necessary.

Conventionally, the identification/classification of living organisms has been carried out using the combination of biochemical and morphological tests. However, these tests often did not provide unequivocal answers to the taxonomic positions of tested organisms.

Recently, the taxonomy of organisms, in particular of bacteria, using rRNA sequences became fashionable. There are many reasons why rRNA molecules have been selected as standard molecules for the molecular taxonomy. They are constituents of all organisms. They exist in abundance, and therefore, can readily be isolated and characterized. For sequence comparison, many conserved regions of rRNA molecules allowed the alignment between distantly related organisms, while variable regions are useful for the distinction of closely related organisms (van de Peer, Y., S. Chapelles, and R. de Wacher. 1996. A quantitative map of nucleotide substitution rates in bacterial rRNA. Nucleic Acids Res. 24: 3381-3391; and Gutell, R. R., N. Larsen, and C. R. Woese. 1994. Lessons from an evolving rRNA: 16S and 23S rRNA structures from a comparative perspective. Microbiol. Rev. 58: 10-26). Furthermore, there is a few evidence for the horizontal transfer of rRNA genes although many other genes are expected to have frequently been transferred from one species to other distantly related species. At present, rRNA sequences are accumulating rapidly and they are accessible via an international database (Ribosomal Database project, http://rdp.life.uiuc.edu/).

However, as is clear from the fact that the evolution speed of rRNA genes is extremely slow, there is little difference in the rRNA sequences between closely related organisms. Therefore, in many times, species belonging to the same genus could not be discriminated by the analysis using rRNA sequences. For example, it is said that bacteria sharing more than 97 % of identity in their 16S rRNA sequences (bacterial small subunit rRNA) might belong to the same species. However, there are cases of bacteria exhibiting more than 99 % identity in their 16S rRNA sequences, and yet belonging to two distinct species as revealed from DNA hybridization analysis. Evidently, due to the slow speed of divergent evolution of the 16S rRNA gene, the resolution of 16S rRNA-based analysis between closely related organisms is lower than that of DNA hybridization analysis (Stackebrandt, E. and Goebel, B. M. 1994. Taxonomic note: a place for DNA-DNA reassociation and 16S rRNA sequence analysis in the present species difinition in bacteriology. *Int*. *J. Syst*. *Bacteriol*. 37: 463-464).

Other problems exist in the rRNA-based phylogenetic analysis. To establish a phylogenetic relationship based on rRNA sequences, these sequences should be aligned. The alignment of rRNA sequences composed from four different constituents (AUCG), however, is not easy, and requires some expertise. The correct sequencing of rRNA genes is also difficult largely due to their highly ordered structure. Furthermore, polymorphism of rRNA was found in some organisms.

In contrast, protein-encoding genes have evolved more rapidly than rRNA-encoding genes, since they allow the so-called neutral mutations that do not cause any amino acid substitutions in their gene products. It is then expected that, by using such protein-encoding genes, more precise phylogenetic analysis can be performed than by using rRNA sequences. Thus, the present inventors have developed and applied a method for the identification/classification or detection/monitoring of organisms using the sequences of *gyrB* genes encoding the B subunit of DNA gyrases (Yamamoto, S. and Harayama, S. 1995. PCR Amplification and Direct Sequencing of *gyrB* Genes with Universal Primers and Their Application to the Detection and Taxonomic Analysis of *Pseudomonas putida* Strains. *Appl*. *Environ*. *Microbiol*. 61: 1104-1109; Yamamoto, S. and Harayama, S. 1996. Phylogenetic Analysis of *Acinetobacter* Strains Based on the Nucleotide Sequences of *gyrB* Genes and on the Amino acid Sequences of Their Products. *Int*. *J*. *Syst. Bacteriol*. 46: 506-511; Yamamoto, S. and Harayama, S. 1998. Phylogenetic relationships of *Pseudomonas putida* strains deduced from the nucleotide sequences of *gyrB*, *rpoD* and 16S rRNA genes. *Int*. *J. Syst*. *Bacteriol*. 48: 813-819; Yamamoto, S., Bouvet, P. J. M. & Harayama, S. 1998. Phylogenetic structures of the genus *Acinetobacter* based on the *gyrB* sequences: Comparison with the grouping by DNA-DNA hybridization. *Int*. *J. Syst*. *Bacteriol*. (in press); Harayama, S. and Yamamoto, S. 1996. Phylogenetic Identification of *Pseudomonas* Strains Based on a Comparison of *gyrB* and *rpoD* Sequences. p. 250-258 in Molecular Biology of Pseudomonads, edited by T. Nakazawa, K. Furukawa, D. Haas, S. Silver. ASM Press, Washington, D.C.; and Watanabe, K., Yamamoto, S., Hino, S. and Harayama, S. 1998. Population dynamics of phenol-degrading bacteria in activated sludge determined by *gyrB* - targeted quantitative PCR. *Appl*. *Environ*. *Microbiol*. 64: 1203-1209).

DNA topoisomerases are essential for the replication, transcription, recombination and repair of DNA and control the level of supercoiling of DNA molecules by cleaving and resealing the phosphodiester bond of DNA. They are classified into type I (EC 5.99. 1.2) and type II (EC 5.99.1.3) according to their enzymatic properties. The DNA gyrase is a type II topoisomerase that is capable of introducing negative supercoiling into a relaxed closed circular DNA molecule. This reaction is coupled with ATP hydrolysis. DNA gyrase can also relax supercoiled DNA without ATP hydrolysis. DNA gyrase consists of two subunit proteins in the quaternary structure of A2B2. The A subunit (GyrA) has a molecular weight of approximately 100 kDa while the B subunit (GyrB) has a molecular weight of either 90 kDa or 70 kDa (Wigley, D. B. 1995. Structure and mechanism of DNA topoisomerases. *Ann*. *Rev*. *Biomol*. *Struct*. 24: 185-208). The genes for DNA gyrase or its isofunctional enzymes should exist in all organisms as they are indispensable for the cell proliferation.

As described above, the present inventors have already developed and applied successfully the method for the identification/c lassification or detection/monitoring of organisms using *gyrB* sequences. In this method, a *gyrB* gene fragment of an organism of interest is amplified by PCR using primers designed from the two amino acid sequences, His-Ala-Gly-Gly-Lys-Phe-Asp and Met-Thr-Asp-Ala-Asp-Val-Asp-Gly, which are highly conserved among the GyrB sequences of many organisms. Subsequently, the amplified fragments are subjected to direct sequencing. Since the *gyrB* genes code for proteins, they have frequently undergone neutral mutations. Thus, the nucleotide sequence of the *gyrB* genes vary considerable even among related organisms. For this reason, the above method has been shown to be effective for discriminating organisms at a level of species of subspecies. The above-mentioned PCR primers designed from the highly conserved amino acid sequences of GyrB were effective in many but not all bacterial species for the PCR amplification of *gyrB*. From DNA of some bacterial species, no PCR amplification was observed using these primers.

Besides, there was another problem associated with these primers. The genes for type IV topoisomerese (*parE*) were also amplified from DNA of some bacterial species by using these primers. Topoisomerase IV (ParE) is a bacterial enzyme that appears to be closely related to DNA gyrase. This enzyme involves in the partition of chromosomes into daughter cells. If a *parE* gene but not *gyrB* gene is amplified from a DNA, and if a phylogenetic analysis is carried out without recognizing that the amplified sequence is *parE* but not *gyrB*, it will bring some confusion to the phylogenetic analysis. To avoid such problem associated with the amplification of paralogous genes, primers which do not amplify *parE* should be developed.

It is an object of the present invention to solve the above-described problems of the primers and to provide a means which enables the identification/classification and detection/monitoring of a wide range of organisms using *gyrB* sequences.

Comparing the amino acid sequence data of GyrB collected by the inventors with those of ParE, the inventors have found a plurality of the amino acid sequences of GyrB which are appropriate for designing PCR primers capable of specifically amplifying *gyrB* genes. By using the newly designed PCR primers in combination with the primers mentioned in the BACKGROUND OF THE INVENTION section, it became possible to determine gyrB sequences more easily and precisely from a wider range of organisms. The present invention has been achieved based on the above-described findings.

The present invention relates to the identification and detection of organisms. Accordingly, the present invention provides a set of primers suitable for the identification of a microorganism, which primer set comprises two primers comprising the nucleotide sequences of:
(i) SEQ ID NO: 33 and SEQ ID NO: 27;
(ii) SEQ ID NO: 25 and SEQ ID NO: 35;
(iii) SEQ ID NO: 29 and SEQ ID NO: 38;
(iv) SEQ ID NO: 41 and SEQ ID NO: 43;
(v) SEQ ID NO: 45 and SEQ ID NO: 48;
(vi) SEQ ID NO: 53 and SEQ ID NO: 62;
(vii) SEQ ID NO: 53 and SEQ ID NO: 58;
(viii) SEQ ID NO: 65 and SEQ ID NO: 50;
(ix) SEQ ID NO: 60 and SEQ ID NO: 31; or
(x) SEQ ID NO: 25 and SEQ ID NO: 43.

The invention also provides:
- a method for detecting a microorganism, which method comprises:
   (i) amplifying DNA from a sample by PCR using a set of primers according to claim 1; and
   (ii) identifying whether the sample comprises a microorganism based on the nucleotide sequence of any amplified DNA fragment;
- a method for identifying a microorganism, which method comprises:
   (i) amplifying DNA from the microorganism by PCR using a set of primers according to claim 1; and
- a method for determining the sequence of a gyrB gene of a microorganism, which method comprises:
   (i) amplifying DNA from the microorganism by PCR using a set of primers according to claim 1; and
   (ii) determining the nucleotide sequence of the gyrB gene of the microorganism based on the sequence of the amplified DNA fragment.

The following drawing illustrates the invention:

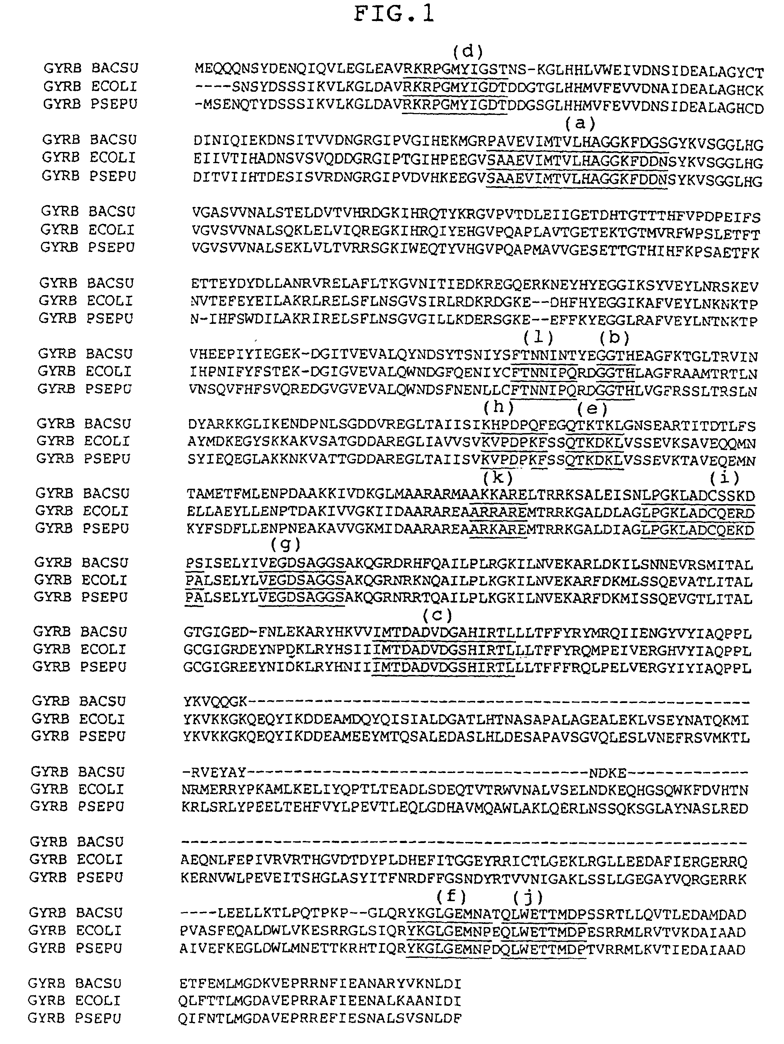

Fig. 1 shows the locational relationship between the amino acid sequences (a) through (l) and the amino acid sequences of GyrB of several organisms.

Hereinbelow, the present invention will be described in detail.

In the present invention, a part of the *gyrB* of an organism of interest is amplified specifically by PCR, and then the nucleotide sequence of the amplified sequences are determined for the taxonomic characterization of the organism.

As a PCR primer, an oligonucleotide may be used which codes for all or a part of one of the following amino acid sequences (a) through (l):
and which functions as a substantial primer. The relationship between the above amino acid sequences (a) through (l) and the amino acid sequences of GyrB from *Bacillus subtilis* 168 strain, *Escherichia coli K-12 strain*
and *Pseudomonas putida* PRS200 strain are shown in Fig. 1.

Most of the amino acid sequences listed (a) through (l) are degenerate, and numerous oligonucleotide sequences can be designed from the listed amino acid sequences. The following amino acid sequences can be enumerated as examples of amino acid sequences to be used for the design of oligonucleotide primers while the following nucleotide sequences can be enumerated as examples of specific primers.

Amino Acid and Oligonucleotides Sequences Corresponding to the Amino Acid Sequence (a):

The amino acid sequence shown in SEQ ID NO: 26, 30, 54, 55, 56 and 57 can be given as the amino acid sequences to be used for the design of oligonucleotide primers.

The nucleotide sequences shown in SEQ ID NO: 25, 29 and 53 can be given as the sequences of primers for the specific amplification of *gyrB.*

Amino Acid and Oligonucleotides Sequences Corresponding to the Amino Acid Sequence (b):

The amino acid sequence shown in SEQ ID NO: 34, 36 and 37 can be given as the amino acid sequences to be used for the design of oligonucleotide primers.

The nucleotide sequences shown in SEQ ID NO: 33 and 35 can be given as the sequences of primers for the specific amplification of *gyrB.*

Amino Acid and Oligonucleotides Sequences Corresponding to the Amino Acid Sequence (c):

The amino acid sequence shown in SEQ ID NO: 28, 32 and 42 can be given as the amino acid sequences to be used for the design of oligonucleotide primers.

The nucleotide sequences shown in SEQ ID NO: 27, 31 and 41 can be given as the sequences of primers for the specific amplification of *gyrB.*

Amino Acid and Oligonucleotides Sequences Corresponding to the Amino Acid Sequence (d):

The amino acid sequence shown in SEQ ID NO: 46 and 47 can be given as the amino acid sequences to be used for the design of oligonucleotide primers.

The nucleotide sequences shown in SEQ ID NO: 45 can be given as the sequences of primers for the specific amplification of *gyrB*.

Amino Acid and Oligonucleotides Sequences Corresponding to the Amino Acid Sequence (e):

The amino acid sequence shown in SEQ ID NO: 39 and 40 can be given as the amino acid sequences to be used for the design of oligonucleotide primers.

The nucleotide sequences shown in SEQ ID NO: 38 can be given as the sequences of primers for the specific amplification of *gyrB*.

Amino Acid and Oligonucleotides Sequences Corresponding to the Amino Acid Sequence (f):

The amino acid sequence shown in SEQ ID NO: 44 can be given as the amino acid sequences to be used for the design of oligonucleotide primers.

The nucleotide sequences shown in SEQ ID NO: 43 can be given as the sequences of primers for the specific amplification of *gyrB*.

Amino Acid and Oligonucleotides Sequences Corresponding to the Amino Acid Sequence (g):

The amino acid sequence shown in SEQ ID NO: 49 can be given as the amino acid sequences to be used for the design of oligonucleotide primers.

The nucleotide sequences shown in SEQ ID NO: 48 can be given as the sequences of primers for the specific amplification of *gyrB.*

Amino Acid and Oligonucleotides Sequences Corresponding to the Amino Acid Sequence (h):

The amino acid sequence shown in SEQ ID NO: 63 and 64 can be given as the amino acid sequences to be used for the design of oligonucleotide primers.

The nucleotide sequences shown in SEQ ID NO: 62 can be given as the sequences of primers for the specific amplification of *gyrB.*

Amino Acid and Oligonucleotides Sequences Corresponding to the Amino Acid Sequence (i):

The amino acid sequence shown in SEQ ID NO: 59 can be given as the amino acid sequences to be used for the design of oligonucleotide primers.

The nucleotide sequences shown in SEQ ID NO: 58 can be given as the sequences of primers for the specific amplification of *gyrB.*

Amino Acid and Oligonucleotides Sequences Corresponding to the Amino Acid Sequence (j):

The amino acid sequence shown in SEQ ID NO: 66, 67 and 68 can be given as the amino acid sequences to be used for the design of oligonucleotide primers.

The nucleotide sequences shown in SEQ ID NO: 65 can be given as the sequences of primers for the specific amplification of *gyrB.*

Amino Acid and Oligonucleotides Sequences Corresponding to the Amino Acid Sequence (k):

The amino acid sequence shown in SEQ ID NO: 51 and 52 can be given as the amino acid sequences to be used for the design of oligonucleotide primers.

The nucleotide sequences shown in SEQ ID NO: 50 can be given as the sequences of primers for the specific amplification of *gyrB.*

Amino Acid and Oligonucleotides Sequences Corresponding to the Amino Acid Sequence (1):

The amino acid sequence shown in SEQ ID NO: 61 can be given as the amino acid sequences to be used for the design of oligonucleotide primers.

The nucleotide sequences shown in SEQ ID NO: 60 can be given as the sequences of primers for the specific amplification of *gyrB.*

Correspondence of each amino acid and oligonucleotide sequence are shown in Table 1.

**Table 1**

| sequence | amino acid | oligonucleotide |
|---|---|---|
| a | SEQ ID NO. 26 | SEQ ID NO. 25 |
| | SEQ ID NO. 30 | SEQ ID NO. 29 |
| | SEQ ID NO. 54,55,56,57 | SEQ ID NO. 53 |
| b | SEQ ID NO. 34 | SEQ ID NO. 33 |
| | SEQ ID NO. 36,37 | SEQ ID NO. 35 |
| c | SEQ ID NO. 28 | SEQ ID NO. 27 |
| | SEQ ID NO. 32 | SEQ ID NO. 31 |
| | SEQ ID NO. 42 | SEQ ID NO. 41 |
| d | SEQ ID NO. 46,47 | SEQ ID NO. 45 |
| e | SEQ ID NO. 39,40 | SEQ ID NO. 38 |
| f | SEQ ID NO. 44 | SEQ ID NO. 43 |
| g | SEQ ID NO. 49 | SEQ ID NO. 48 |
| h | SEQ ID NO. 63,64 | SEQ ID NO. 62 |
| i | SEQ ID NO. 59 | SEQ ID NO. 58 |
| j | SEQ ID NO. 66,67,68 | SEQ ID NO. 65 |
| k | SEQ ID NO. 51,52 | SEQ ID NO. 50 |
| l | SEQ ID NO. 61 | SEQ ID NO. 60 |

The amino acid sequences listed (a) through (l) are not necessarily conserved in all GyrB. Therefore, primers allowing the amplification of *gyrB* should be selected appropriately.

It is possible to directly determine the nucleotide sequence of the amplified PCR product without subcloning by using primers complementary to either the 5-end or the 3-end of the product.

### Examples:

In the following Examples the PCR amplification conditions were as described below.

### PCR amplification conditions:

| | |
|---|---|
| 96 °C 1 min; 48°C 1 min; 72°C 2 min | 3 cycles |
| 96 °C 1 min; 48°C 1 min; 72°C 2 min | 3 cycles |
| 96 °C 1 min; 48°C 1 min; 72°C 2 min | 30 cycles |
| Total | 36 cycles |

| | |
|---|---|
| Primer concentration | 1 µM each |
| dATP | 200 µM each |
| Template DNA | < 1 µg/100 µl |

AmpliTaq™ and the supplied PCR Buffer (Perkin Elmer) were used.

### EXAMPLE 1

A PCR was performed using oligonucleotides represented by the nucleotide sequences shown in SEQ ID NOS: 33 and 27 (corresponding to the amino acid sequences of SEQ ID NOS: 34 and 28, respectively) as primers and DNA from *Chitinophaga pinensis* DSM 2588 strain as a template. The nucleotide sequence of the amplified DNA fragment and the amino acid sequence deduced therefrom are shown in SEQ ID NOS: 5 and 6, respectively.

### EXAMPLE 2

A PCR was performed using oligonucleotides represented by the nucleotide sequences shown in SEQ ID NOS: 25 and 35 (corresponding *to* the amino acid sequences of SEQ ID NO: 26 and SEQ ID NO: 36 or 37, respectively) as primers and DNA from *Flavobacterium aquatile* IAM 12316 strain as a template. The nucleotide sequence of the amplified DNA fragment and the amino acid sequence deduced therefrom are shown in SEQ ID NOS: 7 and 8, respectively.

### EXAMPLE 3

A PCR was performed using oligonucleotides represented by the nucleotide sequences shown in SEQ ID NOS: 29 and 38 (corresponding to the amino acid sequences of SEQ ID NO: 30 and SEQ ID NO: 39 or 40, respectively) as primers and DNA from *Mycobacterium asiaticum* ATCC 25274 strain as a template. The PCR amplification conditions were the same as in Example 1. The nucleotide sequence of the amplified DNA fragment and the amino acid sequence deduced therefrom are shown in SEQ ID NOS: 9 and 10, respectively.

### EXAMPLE 4

A PCR was performed using oligonucleotides represented by the nucleotide sequences shown in SEQ ID NOS: 41 and 43 (corresponding to the amino acid sequences of SEQ ID NOS: 42 and 44, respectively) as primers and DNA from *Cytophaga lytica* IFO 16020 strain as a template. The nucleotide sequence of the amplified DNA fragment and the amino acid sequence deduced therefrom are shown in SEQ ID NOS: 11 and 12, respectively.

### EXAMPLE 5

A PCR was performed using oligonucleotides represented by the nucleotide sequences shown in SEQ ID NOS: 45 and 48 (corresponding to the amino acid sequences of SEQ ID NO: 46 or 47 and SEQ ID NO: 49, respectively) as primers and DNA from *Synechococcus* sp. PCC 6301 strain as a template. The nucleotide sequence of the amplified DNA fragment and the amino acid sequence deduced therefrom are shown in SEQ ID NOS: 13 and 14, respectively.

### EXAMPLE 6

A PCR was performed using oligonucleotides represented by the nucleotide sequences shown in SEQ ID NOS: 53 and 62 (corresponding to the amino acid sequences of SEQ ID NO: 54, 55, 56 or 57 and SEQ ID NO: 63 or 64, respectively) as primers and DNA from *Caulobacter crescentus* ATCC 15252 strain as a template. The nucleotide sequence of the amplified DNA fragment and the amino acid sequence deduced therefrom are shown in SEQ ID NOS: 15 and 16, respectively.

### EXAMPLE 7

A PCR was performed using oligonucleotides represented by the nucleotide sequences shown in SEQ ID NOS: 53 and 58 (corresponding to the amino acid sequences of SEQ ID NO: 54, 55, 56 or 57 and SEQ ID NO: 59, respectively) as primers and DNA from *Pseudomonas putida* ATCC 17484 strain as a template. The nucleotide sequence of the amplified DNA fragment and the amino acid sequence deduced therefrom are shown in SEQ ID NOS: 17 and 18, respectively.

### EXAMPLE 8

A PCR was performed using oligonucleotides represented by the nucleotide sequences shown in SEQ ID NOS: 65 and 50 (corresponding to the amino acid sequences of SEQ ID NO: 66, 67 or 68 and SEQ ID NO: 51 or 52, respectively) as primers and DNA from *Synechococcus* sp. PCC 6301 strain as a template. The nucleotide sequence of the amplified DNA fragment and the amino acid sequence deduced therefrom are shown in SEQ ID NOS: 19 and 20, respectively.

### EXAMPLE 9

A PCR was performed using oligonucleotides represented by the nucleotide sequences shown in SEQ ID NOS: 60 and 31 (corresponding to the amino acid sequences of SEQ ID NOS: 61 and 32, respectively) as primers and DNA from *Caulobacter crescentus* ATCC 15252 strain as a template. The nucleotide sequence of the amplified DNA fragment and the amino acid sequence deduced therefrom are shown in SEQ ID NOS: 21 and 22, respectively,

### EXAMPLE 10

A PCR was performed using oligonucleotides represented by the nucleotide sequences shown in SEQ ID NOS: 25 and 43 (corresponding to the amino acid sequences of SEQ ID NOS: 26 and 44, respectively) as primers and DNA from an unidentified strain MBIC 1544 as a template. The nucleotide sequence of the amplified DNA fragment and the amino acid sequence deduced therefrom are shown in SEQ ID NOS: 23 and 24, respectively.

This nucleotide sequence was compared with the nucleotide sequence database possessed by the applicant. As a result, the unidentified strain MBIC 1544 was identified as *Cytophaga lytica.*

With the nucleotide sequence of *gyrB* determined by the present invention, it is possible to classify or identify an unidentified microorganism strain quickly and accurately. Besides, according to the present invention, PCR primers for monitoring a specific microorganism which are needed in risk assessment in various bioprocesses can be designed easily. Also, the present invention enables highly accurate monitoring of changes in mycelial tufts.

The invention thus allows the determination of the presence or amount of a microorganism in a sample. The invention may be applicable in medical and industrial contexts. For example, in a medical context a sample can be tested for the presence of a microorganism, which may be useful in assessing infection. Therefore, the sample could be serum, blood plasma, or a swab from the eye, ear, mouth, throat, urethra, cervix, vagina, penis or rectum. Alternatively the sample could be a sweep from a culture of bacteria grown on solid or in a liquid media.

In an industrial context, a sample could be tested to determine the amount of a microorganism in a fermentation process. Alternatively a sample could be tested to assess contamination of fermentation broths by unwanted microorganisms. Thus, the sample can be a sample from any bioprocess or fermentation process, where the amount of or presence of a microorganism needs to be ascertained.

### SEQUENCE LISTING

SEQ ID NO: 1
   SEQUENCE LENGTH: 1212
   SEQUENCE TYPE: nucleic acid
   STRANDEDNESS: double
   TOPOLOGY: linear
   MOLECULE TYPE: genomic DNA
   ORIGINAL SOURCE
   ORGANISM: *Bacteroides vulgatus*
   STRAIN: IFO 14291
   SEQUENCE DESCRIPTION 
GAC AAA GGT TCT TAC AAG GTT TCA GGC GGT CTG CAC GGT GTA GGT GTT 48
Asp Lys Gly Ser Tyr Lys Val Ser Gly Gly Leu His Gly Val Gly Val
1               5                   10                  15
TCT TGT GTG AAC GCC TTG TCT ACT CAC ATG ACC ACA CAG GTA TTC CGC 96
Ser Cys Val Asn Ala Leu Ser Thr His Met Thr Thr Gln Val Phe Arg
            20                  25                  30
GGT GGC AAG ATC TAC CAG CAG GAA TAC AGC TGC GGA CAT CCT TTG TAT 144
Gly Gly Lys Ile Tyr Gln Gln Glu Tyr Ser Cys Gly His Pro Leu Tyr
        35                  40                  45
TCT GTA AAA GAA GTA GGA ACA GCT GAT ATT ACC GGA ACA AAA CAG ACT 192
Ser Val Lys Glu Val Gly Thr Ala Asp Ile Thr Gly Thr Lys Gln Thr
    50                  55                  60
TTC TGG CCG GAT GAT ACC ATC TTC ACT GTT ACC GAA TAT AAG TTT GAC 240
Phe Trp Pro Asp Asp Thr Ile Phe Thr Val Thr Glu Tyr Lys Phe Asp
65                  70                  75                  80
ATT CTA CAG GCA CGT ATG CGT GAA TTG GCC TAC TTG AAC AAA GGT ATC 288
Ile Leu Gln Ala Arg Met Arg Glu Leu Ala Tyr Leu Asn Lys Gly Ile
 
                85                  90                  95

ACC ATT TCA CTG ACC GAC CGC CGG ATC AAA GAA GAA GAT GGC AGC TTC 336

Thr Ile Ser Leu Thr Asp Arg Arg Ile Lys Glu Glu Asp Gly Ser Phe

            100                 105                 110

AAG AAA GAA ATA TTC CAT TCG GAC GAA GGA GTG AAA GAG TTT GTA CGT 384

Lys Lys Glu Ile Phe His Ser Asp Glu Gly Val Lys Glu Phe Val Arg

        115                 120                 125

TTC CTG AAC CGT AAC AAC GAA GCG CTG ATT AAT GAT GTC ATT TAT CTG 432

Phe Leu Asn Arg Asn Asn Glu Ala Leu Ile Asn Asp Val Ile Tyr Leu

    130                 135                 140

AAT ACC GAA AAA AAC AAT ACC CCC ATT GAA TGT GCC ATC ATG TAC AAT 480

Asn Thr Glu Lys Asn Asn Thr Pro Ile Glu Cys Ala Ile Met Tyr Asn

145                 150                 155                 160

ACA GGC TAT CGT GAA AGC CTG CAT TCG TAT GTA AAC AAT ATC AAT ACA 528

Thr Gly Tyr Arg Glu Ser Leu His Ser Tyr Val Asn Asn Ile Asn Thr

                165                 170                 175

ATA GAA GGC GGT ACA CAC GAG GCC GGT TTC CGC AGC GCA TTA ACC CGT 576

Ile Glu Gly Gly Thr His Glu Ala Gly Phe Arg Ser Ala Leu Thr Arg

            180                 185                 190

GTA CTG AAG AAA TAT GCG GAA GAT ACC AAA GCA CTG GAA AAA GCA AAA 624

Val Leu Lys Lys Tyr Ala Glu Asp Thr Lys Ala Leu Glu Lys Ala Lys

        195                 200                 205

GTC GAG ATT TCG GGA GAG GAC TTC CGC GAA GGC TTG ATT GCC GTC ATT 672

Val Glu Ile Ser Gly Glu Asp Phe Arg Glu Gly Leu Ile Ala Val Ile

    210                 215                 220

TCA GTG AAA GTA GCC GAG CCG CAG TTC GAA GGA CAG ACC AAG ACC AAG 720

Ser Val Lys Val Ala Glu Pro Gln Phe Glu Gly Gln Thr Lys Thr Lys

225                 230                 235                 240
 
CTG GGC AAC AGC GAA GTG AGT GGT GCC GTG AAC CAA GCT GTA GGC GAA 768
Leu Gly Asn Ser Glu Val Ser Gly Ala Val Asn Gln Ala Val Gly Glu
                245                 250                 255
GCG CTT ACA TAT TAT CTG GAA GAA CAT CCG AAA GAA GCA AAA CAG ATT 816
Ala Leu Thr Tyr Tyr Leu Glu Glu His Pro Lys Glu Ala Lys Gln Ile
            260                 265                 270
GTT GAC AAA GTG ATC CTG GCT GCA ACA GCG CGT ATC GCC GCA CGC AAG 864
Val Asp Lys Val Ile Leu Ala Ala Thr Ala Arg Ile Ala Ala Arg Lys
        275                 280                 285
GCA CGT GAA TCT GTT CAA AGA AAG AGT CCG ATG GGC GGT GGC GGA CTG 912
Ala Arg Glu Ser Val Gln Arg Lys Ser Pro Met Gly Gly Gly Gly Leu
    290                 295                 300
CCG GGC AAA CTG GCC GAC TGC TCG AGC CGT AAT CCG GAG GAA TGT GAA 960
Pro Gly Lys Leu Ala Asp Cys Ser Ser Arg Asn Pro Glu Glu Cys Glu
305                 310                 315                 320
CTA TTC CTG GTC GAG GGT GAC TCG GCA GGT GGT TCT GCC AAG CAA GGA 1008
Leu Phe Leu Val Glu Gly Asp Ser Ala Gly Gly Ser Ala Lys Gln Gly
                325                 330                 335
CGT AGC CGT GCC TTC CAG GCA ATT CTA CCT TTG AGG GGT AAA ATC CTG 1056
Arg Ser Arg Ala Phe Gln Ala Ile Leu Pro Leu Arg Gly Lys Ile Leu
            340                 345                 350
AAT GTG GAA AAA GCG ATG TGG CAC AAG GCT TTT GAA AGC GAT GAG GTC 1104
Asn Val Glu Lys Ala Met Trp His Lys Ala Phe Glu Ser Asp Glu Val
        355                 360                 365
AAT AAT ATC ATC ACC GCC CTG GGT GTC CGT TTC GGT GTG GAC GGA AAT 1152
Asn Asn Ile Ile Thr Ala Leu Gly Val Arg Phe Gly Val Asp Gly Asn
    370                 375                 380
GAT GAC AGC AAA AAA GCG AAC ATC GAC AAG CTG CGT TAT CAC AAA GTG 1200
Asp Asp Ser Lys Lys Ala Asn Ile Asp Lys Leu Arg Tyr His Lys Val
 
385                 390                 395                 400

GTG ATC ATG ACC

Val Ile Met Thr
SEQ ID NO: 2
   SEQUENCE LENGTH: 404
   SEQUENCE TYPE: amino acid
   TOPOLOGY: unknown
   MOLECULE TYPE: protein
   ORIGINAL SOURCE
   ORGANISM: *Bacteroides vulgatus*
   STRAIN: IFO 14291
   SEQUENCE DESCRIPTION 
Asp Lys Gly Ser Tyr Lys Val Ser Gly Gly Leu His Gly Val Gly Val
1               5                   10                  15
Ser Cys Val Asn Ala Leu Ser Thr His Met Thr Thr Gln Val Phe Arg
            20                  25                  30
Gly Gly Lys Ile Tyr Gln Gln Glu Tyr Ser Cys Gly His Pro Leu Tyr
        35                  40                  45
Ser Val Lys Glu Val Gly Thr Ala Asp Ile Thr Gly Thr Lys Gln Thr
    50                  55                  60
Phe Trp Pro Asp Asp Thr Ile Phe Thr Val Thr Glu Tyr Lys Phe Asp
65                  70                  75                  80
Ile Leu Gln Ala Arg Met Arg Glu Leu Ala Tyr Leu Asn Lys Gly Ile
                85                  90                  95
Thr Ile Ser Leu Thr Asp Arg Arg Ile Lys Glu Glu Asp Gly Ser Phe
            100                 105                 110
Lys Lys Glu Ile Phe His Ser Asp Glu Gly Val Lys Glu Phe Val Arg
        115                 120                 125
 
Phe Leu Asn Arg Asn Asn Glu Ala Leu Ile Asn Asp Val Ile Tyr Leu
    130                 135                 140
Asn Thr Glu Lys Asn Asn Thr Pro Ile Glu Cys Ala Ile Met Tyr Asn
145                 150                 155                 160
Thr Gly Tyr Arg Glu Ser Leu His Ser Tyr Val Asn Asn Ile Asn Thr
                165                 170                 175
Ile Glu Gly Gly Thr His Glu Ala Gly Phe Arg Ser Ala Leu Thr Arg
            180                 185                 190
Val Leu Lys Lys Tyr Ala Glu Asp Thr Lys Ala Leu Glu Lys Ala Lys
        195                 200                 205
Val Glu Ile Ser Gly Glu Asp Phe Arg Glu Gly Leu Ile Ala Val Ile
    210                 215                 220
Ser Val Lys Val Ala Glu Pro Gln Phe Glu Gly Gln Thr Lys Thr Lys
225                 230                 235                 240
Leu Gly Asn Ser Glu Val Ser Gly Ala Val Asn Gln Ala Val Gly Glu
                245                 250                 255
Ala Leu Thr Tyr Tyr Leu Glu Glu His Pro Lys Glu Ala Lys Gln Ile
            260                 265                 270
Val Asp Lys Val Ile Leu Ala Ala Thr Ala Arg Ile Ala Ala Arg Lys
        275                 280                 285
Ala Arg Glu Ser Val Gln Arg Lys Ser Pro Met Gly Gly Gly Gly Leu
    290                 295                 300
Pro Gly Lys Leu Ala Asp Cys Ser Ser Arg Asn Pro Glu Glu Cys Glu
305                 310                 315                 320
Leu Phe Leu Val Glu Gly Asp Ser Ala Gly Gly Ser Ala Lys Gln Gly
                325                 330                 335
Arg Ser Arg Ala Phe Gln Ala Ile Leu Pro Leu Arg Gly Lys Ile Leu
            340                 345                 350
Asn Val Glu Lys Ala Met Trp His Lys Ala Phe Glu Ser Asp Glu Val
 
        355                 360                 365
Asn Asn Ile Ile Thr Ala Leu Gly Val Arg Phe Gly Val Asp Gly Asn
    370                 375                 380
Asp Asp Ser Lys Lys Ala Asn Ile Asp Lys Leu Arg Tyr His Lys Val
385                 390                 395                 400
Val Ile Met Thr
SEQ ID NO: 3
   SEQUENCE LENGTH: 1263
   SEQUENCE TYPE: nucleic acid
   STRANDEDNESS: double
   TOPOLOGY: linear
   MOLECULE TYPE: genomic DNA
   ORIGINAL SOURCE
   ORGANISM: *Mycobacterium simiae*
   STRAIN: KPM 1403
   SEQUENCE DESCRIPTION 
GGG GAG AAC AGT GGC TAC ACC GTC AGC GGC GGG TTG CAC GGG GTC GGA 48
Gly Glu Asn Ser Gly Tyr Thr Val Ser Gly Gly Leu His Gly Val Gly
1               5                   10                  15
GTG TCG GTG GTC AAC GCC CTG TCC ACC CGC CTG GAA GTC AAC GTC AAG 96
Val Ser Val Val Asn Ala Leu Ser Thr Arg Leu Glu Val Asn Val Lys
            20                  25                  30
CGT GAC GGC TAT GAG TGG TTC CAG TAC TAC GAC CGG GCG GTG CCC GGC 144
Arg Asp Gly Tyr Glu Trp Phe Gln Tyr Tyr Asp Arg Ala Val Pro Gly
        35                  40                  45
ACC CTC AAG CAA GGC GAG GCG ACC AAG AAG ACC GGC ACC ACG ATC CGG 192
Thr Leu Lys Gln Gly Glu Ala Thr Lys Lys Thr Gly Thr Thr Ile Arg
    50                  55                  60
 
TTC TGG GCC GAT CCT GAG ATC TTC GAA ACC ACC CAG TAC GAC TTC GAG 240
Phe Trp Ala Asp Pro Glu Ile Phe Glu Thr Thr Gln Tyr Asp Phe Glu
65                  70                  75                  80
ACG GTG GCG CGC CGG TTG CAG GAA ATG GCG TTC CTC AAC AAG GGC CTG 288
Thr Val Ala Arg Arg Leu Gln Glu Met Ala Phe Leu Asn Lys Gly Leu
                85                  90                  95
ACC ATC AAC CTC ACC GAC GAA CGT GTC GAG CAG GAC GAG GTG GTC GAT 336
Thr Ile Asn Leu Thr Asp Glu Arg Val Glu Gln Asp Glu Val Val Asp
            100                 105                 110
GAG GTG GTT AGC GAC ACC GCC GAG GCG CCG AAG TCA GCC GAG GAG CAG 384
Glu Val Val Ser Asp Thr Ala Glu Ala Pro Lys Ser Ala Glu Glu Gln
        115                 120                 125
GCG GCC GAA TCG GCC AAG CCG CAC AAG GTC AAG CAC CGC ACG TTC CAC 432
Ala Ala Glu Ser Ala Lys Pro His Lys Val Lys His Arg Thr Phe His
    130                 135                 140
TAC CCG GGT GGG TTG GTG GAT TTC GTC AAG CAC ATC AAT CGC ACC AAA 480
Tyr Pro Gly Gly Leu Val Asp Phe Val Lys His Ile Asn Arg Thr Lys
145                 150                 155                 160
AAC CCG ATC CAG CAG AGC GTC ATC GAC TTC GAC GGC AAA GGA ACC GGG 528
Asn Pro Ile Gln Gln Ser Val Ile Asp Phe Asp Gly Lys Gly Thr Gly
                165                 170                 175
CAC GAA GTC GAG ATC GCG ATG CAG TGG AAC GGT GGT TAT TCG GAG TCG 576
His Glu Val Glu Ile Ala Met Gln Trp Asn Gly Gly Tyr Ser Glu Ser
            180                 185                 190
GTG CAC ACC TTC GCC AAC ACC ATC AAC ACC CAT GAG GGC GGC ACC CAC 624
Val His Thr Phe Ala Asn Thr Ile Asn Thr His Glu Gly Gly Thr His
        195                 200                 205
GAG GAG GGC TTC CGC AGC GCG CTG ACC TCG GTG GTG AAC AAG TAC GCC 672
Glu Glu Gly Phe Arg Ser Ala Leu Thr Ser Val Val Asn Lys Tyr Ala
 
    210                 215                 220

AAA GAC AAG AAG CTG CTC AAG GAC AAG GAT CCC AAC CTC ACC GGC GAC 720

Lys Asp Lys Lys Leu Leu Lys Asp Lys Asp Pro Asn Leu Thr Gly Asp

225                 230                 235                 240

GAC ATC CGA GAA GGG CTG GCC GCG GTG ATC TCC GTG AAG GTC GCC GAG 768

Asp Ile Arg Glu Gly Leu Ala Ala Val Ile Ser Val Lys Val Ala Glu

                245                 250                 255

CCG CAG TTC GAG GGC CAG ACT AAG ACG AAA CTC GGC AAC ACC GAG GTC 816

Pro Gln Phe Glu Gly Gln Thr Lys Thr Lys Leu Gly Asn Thr Glu Val

            260                 265                 270

AAG TCG TTT GTC CAG AAA GTC TGT AAC GAA CAA CTC ACT CAC TGG TTC 864

Lys Ser Phe Val Gln Lys Val Cys Asn Glu Gln Leu Thr His Trp Phe

        275                 280                 285

GAG GCG AAC CCG TCG GAA GCT AAA ACC GTT GTA AAC AAG GCG GTT TCG 912

Glu Ala Asn Pro Ser Glu Ala Lys Thr Val Val Asn Lys Ala Val Ser

    290                 295                 300

TCG GCC CAG GCC CGC ATT GCG GCG CGT AAG GCG CGG GAG TTG GTG CGG 960

Ser Ala Gln Ala Arg Ile Ala Ala Arg Lys Ala Arg Glu Leu Val Arg

305                 310                 315                 320

CGT AAG AGT GCT ACG GAT TTG GGT GGG TTG CCG GGC AAG TTG GCT GAT 1008

Arg Lys Ser Ala Thr Asp Leu Gly Gly Leu Pro Gly Lys Leu Ala Asp

                325                 330                 335

TGC CGC TCG ACG GAT CCG CGG AAG TCT GAG CTG TAT GTG GTG GAA GGT 1056

Cys Arg Ser Thr Asp Pro Arg Lys Ser Glu Leu Tyr Val Val Glu Gly

            340                 345                 350

GAT TCC GCG GGT GGG TCG GCG AAA AGT GGG CGT GAT TCG ATG TTC CAG 1104

Asp Ser Ala Gly Gly Ser Ala Lys Ser Gly Arg Asp Ser Met Phe Gln

        355                 360                 365

GCG ATC TTG CCG CTG CGC GGC AAG ATC ATC AAC GTC GAA AAG GCC CGC 1152
 
Ala Ile Leu Pro Leu Arg Gly Lys Ile Ile Asn Val Glu Lys Ala Arg
    370                 375                 380
ATC GAT CGG GTG CTG AAA AAC ACC GAA GTC CAG GCC ATC ATC ACC GCG 1200
Ile Asp Arg Val Leu Lys Asn Thr Glu Val Gln Ala Ile Ile Thr Ala
385                 390                 395                 400
CTG GGC ACC GGC ATC CAC GAC GAA TTC GAC ATC ACC AAA CTG CGT TAC 1248
Leu Gly Thr Gly Ile His Asp Glu Phe Asp Ile Thr Lys Leu Arg Tyr
                405                 410                 415
CAC AAG ATC GTG TTG 1263
His Lys Ile Val Leu
            420
SEQ ID NO: 4
   SEQUENCE LENGTH: 421
   SEQUENCE TYPE: amino acid
   TOPOLOGY: unknown
   MOLECULE TYPE: protein
   ORIGINAL SOURCE
   ORGANISM: *Mycobacterium simiae*
   STRAIN: KPM 1403
   SEQUENCE DESCRIPTION 
Gly Glu Asn Ser Gly Tyr Thr Val Ser Gly Gly Leu His Gly Val Gly
1               5                   10                  15
Val Ser Val Val Asn Ala Leu Ser Thr Arg Leu Glu Val Asn Val Lys
            20                  25                  30
Arg Asp Gly Tyr Glu Trp Phe Gln Tyr Tyr Asp Arg Ala Val Pro Gly
        35                  40                  45
Thr Leu Lys Gln Gly Glu Ala Thr Lys Lys Thr Gly Thr Thr Ile Arg
    50                  55                  60
 
Phe Trp Ala Asp Pro Glu Ile Phe Glu Thr Thr Gln Tyr Asp Phe Glu
65                  70                  75                  80
Thr Val Ala Arg Arg Leu Gln Glu Met Ala Phe Leu Asn Lys Gly Leu
                85                  90                  95
Thr Ile Asn Leu Thr Asp Glu Arg Val Glu Gln Asp Glu Val Val Asp
            100                 105                 110
Glu Val Val Ser Asp Thr Ala Glu Ala Pro Lys Ser Ala Glu Glu Gln
        115                 120                 125
Ala Ala Glu Ser Ala Lys Pro His Lys Val Lys His Arg Thr Phe His
    130                 135                 140
Tyr Pro Gly Gly Leu Val Asp Phe Val Lys His Ile Asn Arg Thr Lys
145                 150                 155                 160
Asn Pro Ile Gln Gln Ser Val Ile Asp Phe Asp Gly Lys Gly Thr Gly
                165                 170                 175
His Glu Val Glu Ile Ala Met Gln Trp Asn Gly Gly Tyr Ser Glu Ser
            180                 185                 190
Val His Thr Phe Ala Asn Thr Ile Asn Thr His Glu Gly Gly Thr His
        195                 200                 205
Glu Glu Gly Phe Arg Ser Ala Leu Thr Ser Val Val Asn Lys Tyr Ala
    210                 215                 220
Lys Asp Lys Lys Leu Leu Lys Asp Lys Asp Pro Asn Leu Thr Gly Asp
225                 230                 235                 240
Asp Ile Arg Glu Gly Leu Ala Ala Val Ile Ser Val Lys Val Ala Glu
                245                 250                 255
Pro Gln Phe Glu Gly Gln Thr Lys Thr Lys Leu Gly Asn Thr Glu Val
            260                 265                 270
Lys Ser Phe Val Gln Lys Val Cys Asn Glu Gln Leu Thr His Trp Phe
        275                 280                 285
Glu Ala Asn Pro Ser Glu Ala Lys Thr Val Val Asn Lys Ala Val Ser
 
    290                 295                 300

Ser Ala Gln Ala Arg Ile Ala Ala Arg Lys Ala Arg Glu Leu Val Arg

305                 310                 315                 320

Arg Lys Ser Ala Thr Asp Leu Gly Gly Leu Pro Gly Lys Leu Ala Asp

                325                 330                 335

Cys Arg Ser Thr Asp Pro Arg Lys Ser Glu Leu Tyr Val Val Glu Gly

            340                 345                 350

Asp Ser Ala Gly Gly Ser Ala Lys Ser Gly Arg Asp Ser Met Phe Gln

        355                 360                 365

Ala Ile Leu Pro Leu Arg Gly Lys Ile Ile Asn Val Glu Lys Ala Arg

    370                 375                 380

Ile Asp Arg Val Leu Lys Asn Thr Glu Val Gln Ala Ile Ile Thr Ala

385                 390                 395                 400

Leu Gly Thr Gly Ile His Asp Glu Phe Asp Ile Thr Lys Leu Arg Tyr

                405                 410                 415

His Lys Ile Val Leu

            420
SEQ ID NO: 5
   SEQUENCE LENGTH: 660
   SEQUENCE TYPE: nucleic acid
   STRANDEDNESS: double
   TOPOLOGY: linear
   MOLECULE TYPE: genomic DNA
   ORIGINAL SOURCE
   ORGANISM: *Chitinophaga pinensis*
   STRAIN: DSM 2588
   SEQUENCE DESCRIPTION GTA GCA GGC TTC CGC CGT GCG ATA ACC CGT ATC TTC AAG AGC TAT GGT 48 
Val Ala Gly Phe Arg Arg Ala Ile Thr Arg Ile Phe Lys Ser Tyr Gly
1               5                   10                  15
GAT AAG AAC AAA ATG TTC GAA AAA ACC AAG ATC GAA GTA ACA GGT GAT     96
Asp Lys Asn Lys Met Phe Glu Lys Thr Lys Ile Glu Val Thr Gly Asp
            20                  25                  30
GAC TTC CGT GAA GGT CTG AGC GCT ATC ATC AGC GTA AAA GTA CCT GAA    144
Asp Phe Arg Glu Gly Leu Ser Ala Ile Ile Ser Val Lys Val Pro Glu
        35                  40                  45
CCA CAG TTC GAA GGC CAG ACC AAA ACC AAA CTC GGT AAC TCC GAT GTA    192
Pro Gln Phe Glu Gly Gln Thr Lys Thr Lys Leu Gly Asn Ser Asp Val
    50                  55                  60
ATG GGG GTT GTG GAC AGT TCC GTA GCA GCC GTA CTG GAT GCC TAC CTG    240
Met Gly Val Val Asp Ser Ser Val Ala Ala Val Leu Asp Ala Tyr Leu
65                  70                  75                  80
GAA GAA CAT CCC CGC GAA GCC AAG ATC ATT ATC AAT AAA GTG GTA CTG    288
Glu Glu His Pro Arg Glu Ala Lys Ile Ile Ile Asn Lys Val Val Leu
                85                  90                  95
GCA GCA CAG GCG CGT GAA GCA GCC CGT AAA GCA CGC CAG ATG GTA CAG    336
Ala Ala Gln Ala Arg Glu Ala Ala Arg Lys Ala Arg Gln Met Val Gln
            100                 105                 110
CGT AAG AGC GTA CTG AGT GGA AGC GGC TTG CCT GGT AAA CTG GCT GAC    384
Arg Lys Ser Val Leu Ser Gly Ser Gly Leu Pro Gly Lys Leu Ala Asp
        115                 120                 125
TGC TCT GAA AAT GAT CCT GAA AAA TGT GAA CTG TAC CTG GTA GAG GGT    432
Cys Ser Glu Asn Asp Pro Glu Lys Cys Glu Leu Tyr Leu Val Glu Gly
    130                 135                 140
GAC TCC GCA GGT GGT ACG GCT AAA CAA GGA CGT AAC CGT AGC TTC CAG    480
Asp Ser Ala Gly Gly Thr Ala Lys Gln Gly Arg Asn Arg Ser Phe Gln
145                 150                 155                 160
 
GCG ATC CTG CCG CTC AGG GGT AAA ATC CTG AAC GTG GAG AAA GCC ATG 528
Ala Ile Leu Pro Leu Arg Gly Lys Ile Leu Asn Val Glu Lys Ala Met
                165                 170                 175
GAG CAT AAG ATA TAT GAG AAT GAG GAG ATT AAA AAC ATC TTC ACC GCA 576
Glu His Lys Ile Tyr Glu Asn Glu Glu Ile Lys Asn Ile Phe Thr Ala
            180                 185                 190
CTT GGT GTA ACC ATC GGT ACG GAA GAA GAT GAC AAA GCC CTC AAC CTC 624
Leu Gly Val Thr Ile Gly Thr Glu Glu Asp Asp Lys Ala Leu Asn Leu
        195                 200                 205
TCC AAA CTG CGC TAT CAC AAA CTG ATC ATC ATG ACG 660
Ser Lys Leu Arg Tyr His Lys Leu Ile Ile Met Thr
    210                 215                 220
SEQ ID NO: 6
   SEQUENCE LENGTH: 220
   SEQUENCE TYPE: amino acid
   TOPOLOGY: unknown
   MOLECULE TYPE: protein
   ORIGINAL SOURCE
   ORGANISM: *Chitinophaga pinensis*
   STRAIN: DSM 2588
   SEQUENCE DESCRIPTION 
Val Ala Gly Phe Arg Arg Ala Ile Thr Arg Ile Phe Lys Ser Tyr Gly
1               5                   10                  15
Asp Lys Asn Lys Met Phe Glu Lys Thr Lys Ile Glu Val Thr Gly Asp
            20                  25                  30
Asp Phe Arg Glu Gly Leu Ser Ala Ile Ile Ser Val Lys Val Pro Glu
        35                  40                  45
Pro Gln Phe Glu Gly Gln Thr Lys Thr Lys Leu Gly Asn Ser Asp Val
 
    50                  55                  60
Met Gly Val Val Asp Ser Ser Val Ala Ala Val Leu Asp Ala Tyr Leu
65                  70                  75                  80
Glu Glu His Pro Arg Glu Ala Lys Ile Ile Ile Asn Lys Val Val Leu
                85                  90                  95
Ala Ala Gln Ala Arg Glu Ala Ala Arg Lys Ala Arg Gln Met Val Gln
            100                 105                 110
Arg Lys Ser Val Leu Ser Gly Ser Gly Leu Pro Gly Lys Leu Ala Asp
        115                 120                 125
Cys Ser Glu Asn Asp Pro Glu Lys Cys Glu Leu Tyr Leu Val Glu Gly
    130                 135                 140
Asp Ser Ala Gly Gly Thr Ala Lys Gln Gly Arg Asn Arg Ser Phe Gln
145                 150                 155                 160
Ala Ile Leu Pro Leu Arg Gly Lys Ile Leu Asn Val Glu Lys Ala Met
                165                 170                 175
Glu His Lys Ile Tyr Glu Asn Glu Glu Ile Lys Asn Ile Phe Thr Ala
            180                 185                 190
Leu Gly Val Thr Ile Gly Thr Glu Glu Asp Asp Lys Ala Leu Asn Leu
        195                 200                 205
Ser Lys Leu Arg Tyr His Lys Leu Ile Ile Met Thr
    210                 215                 220
SEQ ID NO: 7
   SEQUENCE LENGTH: 537
   SEQUENCE TYPE: nucleic acid
   STRANDEDNESS: double
   TOPOLOGY: linear
   MOLECULE TYPE: genomic DNA
   ORIGINAL SOURCE
   ORGANISM: *Flavobacterium aquatile*
   STRAIN: IAM 12316
   SEQUENCE DESCRIPTION 
GAT AAA GAT TCT TAT AAA GTT TCG GGT GGA CTT CAC GGA GTT GGT GTT 48
Asp Lys Asp Ser Tyr Lys Val Ser Gly Gly Leu His Gly Val Gly Val
1               5                   10                  15
TCT TGC GTT AAT GCA CTT TCT GAT AAC CTA AAA GCA ACC GTT TTT AGA 96
Ser Cys Val Asn Ala Leu Ser Asp Asn Leu Lys Ala Thr Val Phe Arg
            20                  25                  30
GAC GGA AAA GTG TAC GAG CAA GAA TAT GAA AAA GGT AAA GCA ATG TAT 144
Asp Gly Lys Val Tyr Glu Gln Glu Tyr Glu Lys Gly Lys Ala Met Tyr
        35                  40                  45
CCG GTT AAG CAA GTT GGT GAA ACA ACA AAG CGA GGA ACA ATG GTT ACT 192
Pro Val Lys Gln Val Gly Glu Thr Thr Lys Arg Gly Thr Met Val Thr
    50                  55                  60
TTT CAT CCT GAT AAA ACC ATT TTT ACT CAA ACA ATT GAG TAT TCT TAT 240
Phe His Pro Asp Lys Thr Ile Phe Thr Gln Thr Ile Glu Tyr Ser Tyr
65                  70                  75                  80
GAT ACA CTT GCA GCA CGT ATG CGT GAA TTA TCT TTC CTG AAT AAA GGA 288
Asp Thr Leu Ala Ala Arg Met Arg Glu Leu Ser Phe Leu Asn Lys Gly
                85                  90                  95
ATT ACA ATC ACA CTT ACA GAT AAA AGA CAT ACT AAA GAC AAC GGC GAT 336
Ile Thr Ile Thr Leu Thr Asp Lys Arg His Thr Lys Asp Asn Gly Asp
            100                 105                 110
TTT GAA GGT GAA GTT TTT CAT TCT AAA GAA GGG CTT AAA GAA TTC GTT 384
Phe Glu Gly Glu Val Phe His Ser Lys Glu Gly Leu Lys Glu Phe Val
        115                 120                 125
CGA TTT TTA GAT GCT GGT AGA GAA CCA ATT ATT TCT CAC GTA ATA AGC 432
Arg Phe Leu Asp Ala Gly Arg Glu Pro Ile Ile Ser His Val Ile Ser
 
    130                 135                 140

ATG GAG CAC GAA AAA GGA GAA GTT CCT GTT GAG GTT GCT CTT GTT TAC 480

Met Glu His Glu Lys Gly Glu Val Pro Val Glu Val Ala Leu Val Tyr

145                 150                 155                 160

AAT ACA AGT TAC TCC GAA AAT ATT TTC TCT TAC GTA AAT AAT ATT AAC 528

Asn Thr Ser Tyr Ser Glu Asn Ile Phe Ser Tyr Val Asn Asn Ile Asn

                165                 170                 175

ACG CAC GAA 537

Thr His Glu
SEQ ID NO: 8
   SEQUENCE LENGTH: 179
   SEQUENCE TYPE: amino acid
   TOPOLOGY: unknown
   MOLECULE TYPE: protein
   ORIGINAL SOURCE
   ORGANISM: *Flavobacterium aquatile*
   STRAIN: IAM 12316
   SEQUENCE DESCRIPTION 
Asp Lys Asp Ser Tyr Lys Val Ser Gly Gly Leu His Gly Val Gly Val
1               5                   10                  15
Ser Cys Val Asn Ala Leu Ser Asp Asn Leu Lys Ala Thr Val Phe Arg
            20                  25                  30
Asp Gly Lys Val Tyr Glu Gln Glu Tyr Glu Lys Gly Lys Ala Met Tyr
        35                  40                  45
Pro Val Lys Gln Val Gly Glu Thr Thr Lys Arg Gly Thr Met Val Thr
    50                  55                  60
Phe His Pro Asp Lys Thr Ile Phe Thr Gln Thr Ile Glu Tyr Ser Tyr
65                  70                  75                  80
 
Asp Thr Leu Ala Ala Arg Met Arg Glu Leu Ser Phe Leu Asn Lys Gly
                85                  90                  95
Ile Thr Ile Thr Leu Thr Asp Lys Arg His Thr Lys Asp Asn Gly Asp
            100                 105                 110
Phe Glu Gly Glu Val Phe His Ser Lys Glu Gly Leu Lys Glu Phe Val
        115                 120                 125
Arg Phe Leu Asp Ala Gly Arg Glu Pro Ile Ile Ser His Val Ile Ser
    130                 135                 140
Met Glu His Glu Lys Gly Glu Val Pro Val Glu Val Ala Leu Val Tyr
145                 150                 155                 160
Asn Thr Ser Tyr Ser Glu Asn Ile Phe Ser Tyr Val Asn Asn Ile Asn
                165                 170                 175
Thr His Glu
SEQ ID NO: 9
   SEQUENCE LENGTH: 783
   SEQUENCE TYPE: nucleic acid
   STRANDEDNESS: double
   TOPOLOGY: linear
   MOLECULE TYPE: genomic DNA
   ORIGINAL SOURCE
   ORGANISM: *Mycobacterium asiaticum*
   STRAIN: ATCC 25274
   SEQUENCE DESCRIPTION 
GGC GAG AAC AGC GGC TAC ACC GTC AGC GGT GGG TTG CAC GGA GTG GGC 48
Gly Glu Asn Ser Gly Tyr Thr Val Ser Gly Gly Leu His Gly Val Gly
1               5                   10                  15
GTG TCG GTG GTC AAC GCG CTG TCC ACC CGC CTG GAG GTC ACC ATC AAG 96
 
Val Ser Val Val Asn Ala Leu Ser Thr Arg Leu Glu Val Thr Ile Lys
            20                  25                  30
CGC GAC GGG CAC GAG TGG TTT CAG TAC TAC GAC CGC GCC GTG CCC GGA 144
Arg Asp Gly His Glu Trp Phe Gln Tyr Tyr Asp Arg Ala Val Pro Gly
        35                  40                  45
ACC CTC AAG CAG GGC GAG GCC ACC AAG AAG ACC GGA ACC ACG ATC AGG 192
Thr Leu Lys Gln Gly Glu Ala Thr Lys Lys Thr Gly Thr Thr Ile Arg
    50                  55                  60
TTC TGG GCG GAC CCC GAA ATC TTC GAA ACC ACA CAG TAC GAC TTC GAG 240
Phe Trp Ala Asp Pro Glu Ile Phe Glu Thr Thr Gln Tyr Asp Phe Glu
65                  70                  75                  80
ACC GTG GCG CGG CGG CTG CAG GAG ATG GCC TTC CTC AAC AAG GGC CTC 288
Thr Val Ala Arg Arg Leu Gln Glu Met Ala Phe Leu Asn Lys Gly Leu
                85                  90                  95
ACC ATC AAC CTC ACC GAC GAA CGA GTG GAG CAG GAC GAG GTC GTC GAC 336
Thr Ile Asn Leu Thr Asp Glu Arg Val Glu Gln Asp Glu Val Val Asp
            100                 105                 110
GAG GTC GTC AGC GAC ACC GCC GAG GCA CCG AAG TCC GCC GAA GAG AAG 384
Glu Val Val Ser Asp Thr Ala Glu Ala Pro Lys Ser Ala Glu Glu Lys
        115                 120                 125
GCC GCG GAA TCG ACT GCG CCA CAC AAG GTC AAG CAC CGC ACC TTC CAC 432
Ala Ala Glu Ser Thr Ala Pro His Lys Val Lys His Arg Thr Phe His
    130                 135                 140
TAC CCC GGC GGT CTG GTC GAC TTC GTC AAG CAC ATC AAC CGC ACC AAG 480
Tyr Pro Gly Gly Leu Val Asp Phe Val Lys His Ile Asn Arg Thr Lys
145                 150                 155                 160
AGC CCG ATC CAG CAG AGC GTC ATC GAT TTC GAC GGC AAG GGC ACC GGC 528
Ser Pro Ile Gln Gln Ser Val Ile Asp Phe Asp Gly Lys Gly Thr Gly
                165                 170                 175
 
CAC GAG GTC GAG ATC GCC ATG CAG TGG AAC GGC GGC TAC TCG GAG TCC 576
His Glu Val Glu Ile Ala Met Gln Trp Asn Gly Gly Tyr Ser Glu Ser
            180                 185                 190
GTC CAC ACC TTC GCC AAC ACC ATC AAC ACG CAC GAG GGC GGC ACC CAC 624
Val His Thr Phe Ala Asn Thr Ile Asn Thr His Glu Gly Gly Thr His
        195                 200                 205
GAG GAG GGC TTC CGC AGC GCG CTG ACG TCG GTG GTG AAC AAG TAC GCC 672
Glu Glu Gly Phe Arg Ser Ala Leu Thr Ser Val Val Asn Lys Tyr Ala
    210                 215                 220
AAA GAC AAG AAA CTG CTG AAG GAC AAA GAT CCC AAC CTC ACC GGT GAC 720
Lys Asp Lys Lys Leu Leu Lys Asp Lys Asp Pro Asn Leu Thr Gly Asp
225                 230                 235                 240
GAC ATC CGT GAG GGC TTG GCC GCG GTC ATC TCG GTG AAG GTC GCC GAG 768
Asp Ile Arg Glu Gly Leu Ala Ala Val Ile Ser Val Lys Val Ala Glu
                245                 250                 255
CCA CAG TTC GAA GGC 783
Pro Gln Phe Glu Gly
            260
SEQ ID NO: 10
   SEQUENCE LENGTH: 261
   SEQUENCE TYPE: amino acid
   TOPOLOGY: unknown
   MOLECULE TYPE: protein
   ORIGINAL SOURCE
   ORGANISM: *Mycobacterium asiaticum*
   STRAIN: ATCC 25274
   SEQUENCE DESCRIPTION Gly Glu Asn Ser Gly Tyr Thr Val Ser Gly Gly Leu His Gly Val Gly 
1                   5                   10                  15

Val Ser Val Val Asn Ala Leu Ser Thr Arg Leu Glu Val Thr Ile Lys

                20                  25                  30

Arg Asp Gly His Glu Trp Phe Gln Tyr Tyr Asp Arg Ala Val Pro Gly

            35                  40                  45

Thr Leu Lys Gln Gly Glu Ala Thr Lys Lys Thr Gly Thr Thr Ile Arg

        50                  55                  60

Phe Trp Ala Asp Pro Glu Ile Phe Glu Thr Thr Gln Tyr Asp Phe Glu

65                  70                  75                  80

Thr Val Ala Arg Arg Leu Gln Glu Met Ala Phe Leu Asn Lys Gly Leu

                85                  90                  95

Thr Ile Asn Leu Thr Asp Glu Arg Val Glu Gln Asp Glu Val Val Asp

            100                 105                 110

Glu Val Val Ser Asp Thr Ala Glu Ala Pro Lys Ser Ala Glu Glu Lys

        115                 120                 125

Ala Ala Glu Ser Thr Ala Pro His Lys Val Lys His Arg Thr Phe His

    130                 135                 140

Tyr Pro Gly Gly Leu Val Asp Phe Val Lys His Ile Asn Arg Thr Lys

145                 150                 155                 160

Ser Pro Ile Gln Gln Ser Val Ile Asp Phe Asp Gly Lys Gly Thr Gly

                165                 170                 175

His Glu Val Glu Ile Ala Met Gln Trp Asn Gly Gly Tyr Ser Glu Ser

            180                 185                 190

Val His Thr Phe Ala Asn Thr Ile Asn Thr His Glu Gly Gly Thr His

        195                 200                 205

Glu Glu Gly Phe Arg Ser Ala Leu Thr Ser Val Val Asn Lys Tyr Ala

    210                 215                 220

Lys Asp Lys Lys Leu Leu Lys Asp Lys Asp Pro Asn Leu Thr Gly Asp

225                 230                 235                 240
 
Asp Ile Arg Glu Gly Leu Ala Ala Val Ile Ser Val Lys Val Ala Glu
                245                 250                 255
Pro Gln Phe Glu Gly
            260
SEQ ID NO: 11
   SEQUENCE LENGTH: 195
   SEQUENCE TYPE: nucleic acid
   STRANDEDNESS: double
   TOPOLOGY: linear
   MOLECULE TYPE: genomic DNA
   ORIGINAL SOURCE
   ORGANISM: *Cytophaga lytica*
   STRAIN: IFO 16020
   SEQUENCE DESCRIPTION 
AGC CAC ATT GAA ACT TTA ATT CTT ACA TTC TTC TTC CGT TTT ATG CGA 48
Ser His Ile Glu Thr Leu Ile Leu Thr Phe Phe Phe Arg Phe Met Arg
1               5                   10                  15
GAA CTA ATA GAA GGC GGA CAC GTT TAC ATA GCA ACA CCA CCT TTA TAT 96
Glu Leu Ile Glu Gly Gly His Val Tyr Ile Ala Thr Pro Pro Leu Tyr
            20                  25                  30
TTA GTT AAA AAA GGA ACT AAA AAG CGT TAT GCT TGG AAT GAT AAA GAA 144
Leu Val Lys Lys Gly Thr Lys Lys Arg Tyr Ala Trp Asn Asp Lys Glu
        35                  40                  45
CGA GAT GAA ATA GCA GAT AGC TTT AAT GGT AGT GTA GGT ATC CAA AGA 192
Arg Asp Glu Ile Ala Asp Ser Phe Asn Gly Ser Val Gly Ile Gln Arg
    50                  55                  60
TAT 195
Tyr
65
SEQ ID NO: 12
   SEQUENCE LENGTH: 65
   SEQUENCE TYPE: amino acid
   TOPOLOGY: unknown
   MOLECULE TYPE: protein
   ORIGINAL SOURCE
   ORGANISM: *Cytophaga lytica*
   STRAIN: IFO 16020
   SEQUENCE DESCRIPTION 
Ser His Ile Glu Thr Leu Ile Leu Thr Phe Phe Phe Arg Phe Met Arg
1               5                   10                  15
Glu Leu Ile Glu Gly Gly His Val Tyr Ile Ala Thr Pro Pro Leu Tyr
            20                  25                  30
Leu Val Lys Lys Gly Thr Lys Lys Arg Tyr Ala Trp Asn Asp Lys Glu
        35                  40                  45
Arg Asp Glu Ile Ala Asp Ser Phe Asn Gly Ser Val Gly Ile Gln Arg
    50                  55                  60
Tyr
65
SEQ ID NO: 13
   SEQUENCE LENGTH: 1170
   SEQUENCE TYPE: nucleic acid
   STRANDEDNESS: double
   TOPOLOGY: linear
   MOLECULE TYPE: genomic DNA
   ORIGINAL SOURCE
   ORGANISM: *Synechococcus* sp.
   STRAIN: PPC 6301
   SEQUENCE DESCRIPTION 
GTG GTG GAC AAC GCC GTC GAC AAA GCC TTG GCG GGC TAC TGC AAT ACC 48
Val Val Asp Asn Ala Val Asp Lys Ala Leu Ala Gly Tyr Cys Asn Thr
1               5                   10                  15
ATT GAT GTT CGT CTG CTC AAA GAC GGC TCC TGC CAA GTC ACC GAT AAC 96
Ile Asp Val Arg Leu Leu Lys Asp Gly Ser Cys Gln Val Thr Asp Asn
            20                  25                  30
GGT CGC GGC ATT CCC ACA GAT ATT CAC CCC CAA ACC GGG AAG TCT GCT 144
Gly Arg Gly Ile Pro Thr Asp Ile His Pro Gln Thr Gly Lys Ser Ala
        35                  40                  45
CTC GAA ACC GTG CTG ACG ATT CTG CAC GCG GGC GGC AAG TTT GGC GGT 192
Leu Glu Thr Val Leu Thr Ile Leu His Ala Gly Gly Lys Phe Gly Gly
    50                  55                  60
GGC GGT TAT AAG GTG TCG GGG GGT CTG CAC GGC GTC GGT GTG TCT GTC 240
Gly Gly Tyr Lys Val Ser Gly Gly Leu His Gly Val Gly Val Ser Val
65                  70                  75                  80
GTC AAC GCC CTC TCA GAA TAT GTC GAA GTC ACC GTG TGG CGG GAA GGC 288
Val Asn Ala Leu Ser Glu Tyr Val Glu Val Thr Val Trp Arg Glu Gly
                85                  90                  95
AAA ACC CAC CAA CAG CGC TTT GAA CAG GGC AAC CCG ATC GGG GAG TTG 336
Lys Thr His Gln Gln Arg Phe Glu Gln Gly Asn Pro Ile Gly Glu Leu
            100                 105                 110
CAA GTT GCC CCG GAT GCC GAC GAT CGC CGC GGG ACA CAA GTT CGT TTC 384
Gln Val Ala Pro Asp Ala Asp Asp Arg Arg Gly Thr Gln Val Arg Phe
        115                 120                 125
AAA CCA GAC GCC ACG ATC TTT TCT GAA ACA ACC GAG TTC GAT TAC GGC 432
 
Lys Pro Asp Ala Thr Ile Phe Ser Glu Thr Thr Glu Phe Asp Tyr Gly
    130                 135                 140
ACC CTA GCA AGC CGA TTG AAG GAG CTA GCC TAT CTG AAT GCG GGC GTC 480
Thr Leu Ala Ser Arg Leu Lys Glu Leu Ala Tyr Leu Asn Ala Gly Val
145                 150                 155                 160
CGC ATC GAC TTT ACC GAT GAG CGG CTG CAG CTC ACC AAG AAT CAC GAG 528
Arg Ile Asp Phe Thr Asp Glu Arg Leu Gln Leu Thr Lys Asn His Glu
                165                 170                 175
CCC CAT CAA GAA ACC TAT TAC TTT GAA GGC GGT ATT CGC GAA TAC GTC 576
Pro His Gln Glu Thr Tyr Tyr Phe Glu Gly Gly Ile Arg Glu Tyr Val
            180                 185                 190
GCC TAC ATG AAT ACC GAT AAA CAG GCG CTG CAC TCA GAG ATT ATC TTT 624
Ala Tyr Met Asn Thr Asp Lys Gln Ala Leu His Ser Glu Ile Ile Phe
        195                 200                 205
GTG CAA TCC GAA AAA GAT GGC GTC CAA GTT GAA GCT GCA TTG CAA TGG 672
Val Gln Ser Glu Lys Asp Gly Val Gln Val Glu Ala Ala Leu Gln Trp
    210                 215                 220
TGC GTT GAC GCC TAC AGC GAC AAC ATT CTG GGC TTT GCC AAC AAC ATC 720
Cys Val Asp Ala Tyr Ser Asp Asn Ile Leu Gly Phe Ala Asn Asn Ile
225                 230                 235                 240
CGC ACG ATT GAC GGC GGC ACC CAT ATT GAG GGG CTC AAA ACT GTT CTG 768
Arg Thr Ile Asp Gly Gly Thr His Ile Glu Gly Leu Lys Thr Val Leu
                245                 250                 255
ACG CGG ACG ATG AAC ACG ATC GCC CGC AAA CGG AAT AAA CGC AAG GAT 816
Thr Arg Thr Met Asn Thr Ile Ala Arg Lys Arg Asn Lys Arg Lys Asp
            260                 265                 270
GCC GAC AAT AAC CTG TCG GGC GAG AAT ATT CGC GAA GGG TTA ACA GCG 864
Ala Asp Asn Asn Leu Ser Gly Glu Asn Ile Arg Glu Gly Leu Thr Ala
        275                 280                 285
 
ATC GTT TCG GTC AAA GTT CCG GAT CCG GAA TTT GAA GGG CAA ACC AAA 912
Ile Val Ser Val Lys Val Pro Asp Pro Glu Phe Glu Gly Gln Thr Lys
    290                 295                 300
ACA AAG CTC GGC AAT ACC GAA GTT CGC GGC ATC GTC GAT ACG CTC GTG 960
Thr Lys Leu Gly Asn Thr Glu Val Arg Gly Ile Val Asp Thr Leu Val
305                 310                 315                 320
GGC GAA ACG TTG ACG GAA TAT CTG GAA TTC CAT CCC AGC GTT GCC GAT 1008
Gly Glu Thr Leu Thr Glu Tyr Leu Glu Phe His Pro Ser Val Ala Asp
                325                 330                 335
TTG ATC CTC GAA AAA GCG ATT CAA GCC TTT AAT GCG GCT GAG GCA GCG 1056
Leu Ile Leu Glu Lys Ala Ile Gln Ala Phe Asn Ala Ala Glu Ala Ala
            340                 345                 350
CGA CGG GCA CGG GAA TTG GTG CGT CGC AAA TCA GTG CTG GAA TCT TCG 1104
Arg Arg Ala Arg Glu Leu Val Arg Arg Lys Ser Val Leu Glu Ser Ser
        355                 360                 365
ACA TTG CCC GGT AAA TTA GCA GAC TGT TCC AGT CGC GAT CCC GGT GAA 1152
Thr Leu Pro Gly Lys Leu Ala Asp Cys Ser Ser Arg Asp Pro Gly Glu
    370                 375                 380
TCT GAA ATC TTC ATC GTG 1170
Ser Glu Ile Phe Ile Val
385                 390
SEQ ID NO: 14
   SEQUENCE LENGTH: 390
   SEQUENCE TYPE: amino acid
   TOPOLOGY: unknown
   MOLECULE TYPE: protein
   ORIGINAL SOURCE
   ORGANISM: *Synechococcus* sp.
   STRAIN: PCC 6301
   SEQUENCE DESCRIPTION 
Val Val Asp Asn Ala Val Asp Lys Ala Leu Ala Gly Tyr Cys Asn Thr
1               5                   10                  15
Ile Asp Val Arg Leu Leu Lys Asp Gly Ser Cys Gln Val Thr Asp Asn
            20                  25                  30
Gly Arg Gly Ile Pro Thr Asp Ile His Pro Gln Thr Gly Lys Ser Ala
        35                  40                  45
Leu Glu Thr Val Leu Thr Ile Leu His Ala Gly Gly Lys Phe Gly Gly
    50                  55                  60
Gly Gly Tyr Lys Val Ser Gly Gly Leu His Gly Val Gly Val Ser Val
65                  70                  75                  80
Val Asn Ala Leu Ser Glu Tyr Val Glu Val Thr Val Trp Arg Glu Gly
                85                  90                  95
Lys Thr His Gln Gln Arg Phe Glu Gln Gly Asn Pro Ile Gly Glu Leu
            100                 105                 110
Gln Val Ala Pro Asp Ala Asp Asp Arg Arg Gly Thr Gln Val Arg Phe
        115                 120                 125
Lys Pro Asp Ala Thr Ile Phe Ser Glu Thr Thr Glu Phe Asp Tyr Gly
    130                 135                 140
Thr Leu Ala Ser Arg Leu Lys Glu Leu Ala Tyr Leu Asn Ala Gly Val
145                 150                 155                 160
Arg Ile Asp Phe Thr Asp Glu Arg Leu Gln Leu Thr Lys Asn His Glu
                165                 170                 175
Pro His Gln Glu Thr Tyr Tyr Phe Glu Gly Gly Ile Arg Glu Tyr Val
            180                 185                 190
Ala Tyr Met Asn Thr Asp Lys Gln Ala Leu His Ser Glu Ile Ile Phe
        195                 200                 205
Val Gln Ser Glu Lys Asp Gly Val Gln Val Glu Ala Ala Leu Gln Trp
 
    210                 215                 220
Cys Val Asp Ala Tyr Ser Asp Asn Ile Leu Gly Phe Ala Asn Asn Ile
225                 230                 235                 240
Arg Thr Ile Asp Gly Gly Thr His Ile Glu Gly Leu Lys Thr Val Leu
                245                 250                 255
Thr Arg Thr Met Asn Thr Ile Ala Arg Lys Arg Asn Lys Arg Lys Asp
            260                 265                 270
Ala Asp Asn Asn Leu Ser Gly Glu Asn Ile Arg Glu Gly Leu Thr Ala
        275                 280                 285
Ile Val Ser Val Lys Val Pro Asp Pro Glu Phe Glu Gly Gln Thr Lys
    290                 295                 300
Thr Lys Leu Gly Asn Thr Glu Val Arg Gly Ile Val Asp Thr Leu Val
305                 310                 315                 320
Gly Glu Thr Leu Thr Glu Tyr Leu Glu Phe His Pro Ser Val Ala Asp
                325                 330                 335
Leu Ile Leu Glu Lys Ala Ile Gln Ala Phe Asn Ala Ala Glu Ala Ala
            340                 345                 350
Arg Arg Ala Arg Glu Leu Val Arg Arg Lys Ser Val Leu Glu Ser Ser
        355                 360                 365
Thr Leu Pro Gly Lys Leu Ala Asp Cys Ser Ser Arg Asp Pro Gly Glu
    370                 375                 380
Ser Glu Ile Phe Ile Val
385                 390
SEQ ID NO: 15
   SEQUENCE LENGTH: 696
   SEQUENCE TYPE: nucleic acid
   STRANDEDNESS: double
   TOPOLOGY: linear
   MOLECULE TYPE: genomic DNA
   ORIGINAL SOURCE
   ORGANISM: *Caulobacter crescentus*
   STRAIN: ATCC 15252
   SEQUENCE DESCRIPTION 
CAG AAC AGC TAC AAG GTC TCG GGC GGT CTG CAC GGC GTG GGC GTC TCG 48
Gln Asn Ser Tyr Lys Val Ser Gly Gly Leu His Gly Val Gly Val Ser
1               5                   10                  15
GTC GTG AAC GCC CTG TCG GAT TGG CTG GAG CTG CTG ATC CAC CGC AAC 96
Val Val Asn Ala Leu Ser Asp Trp Leu Glu Leu Leu Ile His Arg Asn
            20                  25                  30
GGC AAG GTC CAC CAG ATG CGC TTC GAG CGC GGC GAC GCG GTC ACC TCG 144
Gly Lys Val His Gln Met Arg Phe Glu Arg Gly Asp Ala Val Thr Ser
        35                  40                  45
CTG AAG GTC ACC GGC GAC TCG CCC GTG CGG ACC GAG GGC CCC AAG GCC 192
Leu Lys Val Thr Gly Asp Ser Pro Val Arg Thr Glu Gly Pro Lys Ala
    50                  55                  60
GGC GAG ACC CTG ACC GGT ACG GAA GTT ACG TTC TTT CCG TCG AAG GAC 240
Gly Glu Thr Leu Thr Gly Thr Glu Val Thr Phe Phe Pro Ser Lys Asp
65                  70                  75                  80
ACC TTC GCC TTC ATC GAA TTC GAC CGG AAG ACG CTG GAG CAC CGC CTG 288
Thr Phe Ala Phe Ile Glu Phe Asp Arg Lys Thr Leu Glu His Arg Leu
                85                  90                  95
CGC GAG CTG GCC TTC CTG AAC TCG GGC GTG ACG ATC TGG TTC AAG GAC 336
Arg Glu Leu Ala Phe Leu Asn Ser Gly Val Thr Ile Trp Phe Lys Asp
            100                 105                 110
CAT CGC GAC GTC GAG CCG TGG GAA GAG AAG CTG TTC TAC GAG GGC GGC 384
His Arg Asp Val Glu Pro Trp Glu Glu Lys Leu Phe Tyr Glu Gly Gly
        115                 120                 125
 
ATC GAG GCC TTC GTG CGC CAC CTC GAC AAG GCC AAG ACG CCG CTG CTG 432

Ile Glu Ala Phe Val Arg His Leu Asp Lys Ala Lys Thr Pro Leu Leu

    130                 135                 140

AAG GCC CCG ATC GCC GTC AAG GGC GTC AAG GAC AAG GTC GAG ATC GAC 480

Lys Ala Pro Ile Ala Val Lys Gly Val Lys Asp Lys Val Glu Ile Asp

145                 150                 155                 160

CTG GCC CTG TGG TGG AAC GAC AGC TAC CAC GAG CAG ATG CTG TGC TTC 528

Leu Ala Leu Trp Trp Asn Asp Ser Tyr His Glu Gln Met Leu Cys Phe

                165                 170                 175

ACC AAC AAC ATC CCG CAG CGG GAT GGC GGC ACG CAC CTG TCG GCC TTT 576

Thr Asn Asn Ile Pro Gln Arg Asp Gly Gly Thr His Leu Ser Ala Phe

            180                 185                 190

CGC GCG GCC CTG ACC CGG ATC ATC ACC AGC TAC GCC GAG AGC TCC GGC 624

Arg Ala Ala Leu Thr Arg Ile Ile Thr Ser Tyr Ala Glu Ser Ser Gly

        195                 200                 205

ATC CTG AAG AAG GAA AAG GTC AGC CTG GGC GGC GAA GAC AGC CGC GAG 672

Ile Leu Lys Lys Glu Lys Val Ser Leu Gly Gly Glu Asp Ser Arg Glu

    210                 215                 220

GGC CTG ACC TGC GTG CTG TCG GTC 696

Gly Leu Thr Cys Val Leu Ser Val

225                 230
SEQ ID NO: 16
   SEQUENCE LENGTH: 232
   SEQUENCE TYPE: amino acid
   TOPOLOGY: unknown
   MOLECULE TYPE: protein
   ORIGINAL SOURCE
   ORGANISM: *Caulobacter crescentus*
   STRAIN: ATCC 15252
   SEQUENCE DESCRIPTION 
Gln Asn Ser Tyr Lys Val Ser Gly Gly Leu His Gly Val Gly Val Ser
1               5                   10                  15
Val Val Asn Ala Leu Ser Asp Trp Leu Glu Leu Leu Ile His Arg Asn
            20                  25                  30
Gly Lys Val His Gln Met Arg Phe Glu Arg Gly Asp Ala Val Thr Ser
        35                  40                  45
Leu Lys Val Thr Gly Asp Ser Pro Val Arg Thr Glu Gly Pro Lys Ala
    50                  55                  60
Gly Glu Thr Leu Thr Gly Thr Glu Val Thr Phe Phe Pro Ser Lys Asp
65                  70                  75                  80
Thr Phe Ala Phe Ile Glu Phe Asp Arg Lys Thr Leu Glu His Arg Leu
                85                  90                  95
Arg Glu Leu Ala Phe Leu Asn Ser Gly Val Thr Ile Trp Phe Lys Asp
            100                 105                 110
His Arg Asp Val Glu Pro Trp Glu Glu Lys Leu Phe Tyr Glu Gly Gly
        115                 120                 125
Ile Glu Ala Phe Val Arg His Leu Asp Lys Ala Lys Thr Pro Leu Leu
    130                 135                 140
Lys Ala Pro Ile Ala Val Lys Gly Val Lys Asp Lys Val Glu Ile Asp
145                 150                 155                 160
Leu Ala Leu Trp Trp Asn Asp Ser Tyr His Glu Gln Met Leu Cys Phe
                165                 170                 175
Thr Asn Asn Ile Pro Gln Arg Asp Gly Gly Thr His Leu Ser Ala Phe
            180                 185                 190
Arg Ala Ala Leu Thr Arg Ile Ile Thr Ser Tyr Ala Glu Ser Ser Gly
        195                 200                 205
Ile Leu Lys Lys Glu Lys Val Ser Leu Gly Gly Glu Asp Ser Arg Glu
 
    210                 215                 220
Gly Leu Thr Cys Val Leu Ser Val
225                 230
SEQ ID NO: 17
   SEQUENCE LENGTH: 888
   SEQUENCE TYPE: nucleic acid
   STRANDEDNESS: double
   TOPOLOGY: linear
   MOLECULE TYPE: genomic DNA
   ORIGINAL SOURCE
   ORGANISM: *Pseudomonas putida*
   STRAIN: ATCC 17484
   SEQUENCE DESCRIPTION 
GGC GGC CTG CAC GGT GTA GGC GTG TCG GTA GTG AAC GCA CTG TCT GAA 48
Gly Gly Leu His Gly Val Gly Val Ser Val Val Asn Ala Leu Ser Glu
1               5                   10                  15
GAG CTC GTC CTC ACC GTT CGC CGT AGC GGC AAG ATC TGG GAA CAG ACC 96
Glu Leu Val Leu Thr Val Arg Arg Ser Gly Lys Ile Trp Glu Gln Thr
            20                  25                  30
TAC GTC CAT GGT GTT CCG CAG GAA CCG ATG AAG ATC GTT GGC GAC AGC 144
Tyr Val His Gly Val Pro Gln Glu Pro Met Lys Ile Val Gly Asp Ser
        35                  40                  45
GAA ACC ACC GGC ACC CAG ATC CAC TTC AAG GCT TCC AGC GAA ACC TTC 192
Glu Thr Thr Gly Thr Gln Ile His Phe Lys Ala Ser Ser Glu Thr Phe
    50                  55                  60
AAG AAC ATC CAC TTC AGC TGG GAC ATC CTG GCC AAG CGG ATT CGT GAA 240
Lys Asn Ile His Phe Ser Trp Asp Ile Leu Ala Lys Arg Ile Arg Glu
65                  70                  75                  80
 
CTG TCC TTC CTC AAC TCC GGT GTC GGC ATC GTC CTC AAG GAT GAG CGC 288
Leu Ser Phe Leu Asn Ser Gly Val Gly Ile Val Leu Lys Asp Glu Arg
                85                  90                  95
AGC GGC AAG GAA GAA CTG TTC AAG TAC GAA GGC GGC TTG CGC GCG TTC 336
Ser Gly Lys Glu Glu Leu Phe Lys Tyr Glu Gly Gly Leu Arg Ala Phe
            100                 105                 110
GTT GAA TAC CTG AAC ACC AAC AAG ACC CCG GTC AAC CAG GTG TTC CAT 384
Val Glu Tyr Leu Asn Thr Asn Lys Thr Pro Val Asn Gln Val Phe His
        115                 120                 125
TTC AAC ATC CAG CGC GAA GAC GGC ATC GGC GTA GAA ATC GCC CTG CAG 432
Phe Asn Ile Gln Arg Glu Asp Gly Ile Gly Val Glu Ile Ala Leu Gln
    130                 135                 140
TGG AAC GAC AGC TTC AAC GAG AAC CTG TTG TGC TTC ACC AAC AAC ATT 480
Trp Asn Asp Ser Phe Asn Glu Asn Leu Leu Cys Phe Thr Asn Asn Ile
145                 150                 155                 160
CCG CAG CGC GAT GGC GGT ACT CAC CTG GTG GGT TTC CGT TCC GCC CTG 528
Pro Gln Arg Asp Gly Gly Thr His Leu Val Gly Phe Arg Ser Ala Leu
                165                 170                 175
ACG CGT AAC CTC AAT ACG TAT ATC GAA GCC GAA GGC CTG GCG AAG AAG 576
Thr Arg Asn Leu Asn Thr Tyr Ile Glu Ala Glu Gly Leu Ala Lys Lys
            180                 185                 190
CAC AAG GTC GCG ACC ACC GGT GAC GAT GCC CGT GAA GGC CTG GCC GCG 624
His Lys Val Ala Thr Thr Gly Asp Asp Ala Arg Glu Gly Leu Ala Ala
        195                 200                 205
ATC ATT TCG GTA AAA GTG CCG GAT CCG AAG TTC AGC TCC CAG ACC AAG 672
Ile Ile Ser Val Lys Val Pro Asp Pro Lys Phe Ser Ser Gln Thr Lys
    210                 215                 220
GAC AAG CTG GTT TCT TCC GAA GTG AAG ACC GCG GTC GAA CAG GAA ATG 720
Asp Lys Leu Val Ser Ser Glu Val Lys Thr Ala Val Glu Gln Glu Met
 
225                 230                 235                 240
GGC AAG TAC TTC TCC GAC TTC CTG CTG GAA AAC CCG AAC GAA GCC AAG 768
Gly Lys Tyr Phe Ser Asp Phe Leu Leu Glu Asn Pro Asn Glu Ala Lys
                245                 250                 255
CTG GTT GTC GGC AAG ATG ATC GAC GCG GCA CGT GCT CGT GAA GCG GCG 816
Leu Val Val Gly Lys Met Ile Asp Ala Ala Arg Ala Arg Glu Ala Ala
            260                 265                 270
CGC AAG ACC CGT GAG ATG ACC CGC CGC AAA GGC GCG CTG GAC ATC GCC 864
Arg Lys Thr Arg Glu Met Thr Arg Arg Lys Gly Ala Leu Asp Ile Ala
        275                 280                 285
GGC CTG CCG GGC AAA CTG GCT GAC 888
Gly Leu Pro Gly Lys Leu Ala Asp
    290                 295
SEQ ID NO: 18
   SEQUENCE LENGTH: 296
   SEQUENCE TYPE: amino acid
   TOPOLOGY: unknown
   MOLECULE TYPE: protein
   ORIGINAL SOURCE
   ORGANISM: *Pseudomonas putida*
   STRAIN: ATCC 17484
   SEQUENCE DESCRIPTION 
Gly Gly Leu His Gly Val Gly Val Ser Val Val Asn Ala Leu Ser Glu
1               5                   10                  15
Glu Leu Val Leu Thr Val Arg Arg Ser Gly Lys Ile Trp Glu Gln Thr
            20                  25                  30
Tyr Val His Gly Val Pro Gln Glu Pro Met Lys Ile Val Gly Asp Ser
        35                  40                  45
 
Glu Thr Thr Gly Thr Gln Ile His Phe Lys Ala Ser Ser Glu Thr Phe
    50                  55                  60
Lys Asn Ile His Phe Ser Trp Asp Ile Leu Ala Lys Arg Ile Arg Glu
65                  70                  75                  80
Leu Ser Phe Leu Asn Ser Gly Val Gly Ile Val Leu Lys Asp Glu Arg
                85                  90                  95
Ser Gly Lys Glu Glu Leu Phe Lys Tyr Glu Gly Gly Leu Arg Ala Phe
            100                 105                 110
Val Glu Tyr Leu Asn Thr Asn Lys Thr Pro Val Asn Gln Val Phe His
        115                 120                 125
Phe Asn Ile Gln Arg Glu Asp Gly Ile Gly Val Glu Ile Ala Leu Gln
    130                 135                 140
Trp Asn Asp Ser Phe Asn Glu Asn Leu Leu Cys Phe Thr Asn Asn Ile
145                 150                 155                 160
Pro Gln Arg Asp Gly Gly Thr His Leu Val Gly Phe Arg Ser Ala Leu
                165                 170                 175
Thr Arg Asn Leu Asn Thr Tyr Ile Glu Ala Glu Gly Leu Ala Lys Lys
            180                 185                 190
His Lys Val Ala Thr Thr Gly Asp Asp Ala Arg Glu Gly Leu Ala Ala
        195                 200                 205
Ile Ile Ser Val Lys Val Pro Asp Pro Lys Phe Ser Ser Gln Thr Lys
    210                 215                 220
Asp Lys Leu Val Ser Ser Glu Val Lys Thr Ala Val Glu Gln Glu Met
225                 230                 235                 240
Gly Lys Tyr Phe Ser Asp Phe Leu Leu Glu Asn Pro Asn Glu Ala Lys
                245                 250                 255
Leu Val Val Gly Lys Met Ile Asp Ala Ala Arg Ala Arg Glu Ala Ala
            260                 265                 270
Arg Lys Thr Arg Glu Met Thr Arg Arg Lys Gly Ala Leu Asp Ile Ala
 
        275                 280                 285
Gly Leu Pro Gly Lys Leu Ala Asp
    290                 295
SEQ ID NO: 19
   SEQUENCE LENGTH: 531
   SEQUENCE TYPE: nucleic acid
   STRANDEDNESS: double
   TOPOLOGY: linear
   MOLECULE TYPE: genomic DNA
   ORIGINAL SOURCE
   ORGANISM: *Synechococcus* sp.
   STRAIN: PCC 6301
   SEQUENCE DESCRIPTION 
TTG GTG CGT CGC AAA TCA GTG CTG GAA TCT TCG ACA TTG CCC GGT AAA 48
Leu Val Arg Arg Lys Ser Val Leu Glu Ser Ser Thr Leu Pro Gly Lys
1               5                   10                  15
TTA GCA GAC TGT TCC AGT CGC GAT CCC GGT GAA TCT GAA ATC TTC ATC 96
Leu Ala Asp Cys Ser Ser Arg Asp Pro Gly Glu Ser Glu Ile Phe Ile
            20                  25                  30
GTG GAA GGG GAT TCG GCA GGT GGC AGT GCT AAA CAG GGG CGC GAT CGC 144
Val Glu Gly Asp Ser Ala Gly Gly Ser Ala Lys Gln Gly Arg Asp Arg
        35                  40                  45
CGC TTC CAA GCC ATC CTG CCT CTG CGC GGC AAA ATC CTC AAC ATC GAG 192
Arg Phe Gln Ala Ile Leu Pro Leu Arg Gly Lys Ile Leu Asn Ile Glu
    50                  55                  60
AAA ACG GAC GAT GCC AAA ATC TAC AAA AAC ACT GAG ATC CAA GCC CTG 240
Lys Thr Asp Asp Ala Lys Ile Tyr Lys Asn Thr Glu Ile Gln Ala Leu
65                  70                  75                  80
 
ATT ACA GCG CTG GGC CTC GGA ATT AAA GGG GAG GAA TTT GAT GCT TCC 288
Ile Thr Ala Leu Gly Leu Gly Ile Lys Gly Glu Glu Phe Asp Ala Ser
                85                  90                  95
CAA CTG CGC TAC CAC CGT ATT GTG ATC ATG ACT GAC GCG GAC GTC GAT 336
Gln Leu Arg Tyr His Arg Ile Val Ile Met Thr Asp Ala Asp Val Asp
            100                 105                 110
GGT GCG CAC ATC CGT ACC CTC TTG CTC ACC TTC TTC TAT CGC TAT CAG 384
Gly Ala His Ile Arg Thr Leu Leu Leu Thr Phe Phe Tyr Arg Tyr Gln
        115                 120                 125
CGA TCG CTG CTG GAG CAG GGC TAC ATG TAC ATT GCC TGC CCG CCG CTG 432
Arg Ser Leu Leu Glu Gln Gly Tyr Met Tyr Ile Ala Cys Pro Pro Leu
    130                 135                 140
TAC AAG TTG GAG CGG GGA CGT AAT CAC TAC TAT TGC TAC AAC GAA CGC 480
Tyr Lys Leu Glu Arg Gly Arg Asn His Tyr Tyr Cys Tyr Asn Glu Arg
145                 150                 155                 160
GAA CTG CAG GAA CGG ATT GCG ACG TTC CCT GAA AAC GCC AAC TAT ACG 528
Glu Leu Gln Glu Arg Ile Ala Thr Phe Pro Glu Asn Ala Asn Tyr Thr
                165                 170                 175
ATT 531
Ile
SEQ ID NO: 20
   SEQUENCE LENGTH: 177
   SEQUENCE TYPE: amino acid
   TOPOLOGY: unknown
   MOLECULE TYPE: protein
   ORIGINAL SOURCE
   ORGANISM: *Synechococcus* sp.
   STRAIN: PCC 6301
   SEQUENCE DESCRIPTION 
Leu Val Arg Arg Lys Ser Val Leu Glu Ser Ser Thr Leu Pro Gly Lys
1               5                   10                  15
Leu Ala Asp Cys Ser Ser Arg Asp Pro Gly Glu Ser Glu Ile Phe Ile
            20                  25                  30
Val Glu Gly Asp Ser Ala Gly Gly Ser Ala Lys Gln Gly Arg Asp Arg
        35                  40                  45
Arg Phe Gln Ala Ile Leu Pro Leu Arg Gly Lys Ile Leu Asn Ile Glu
    50                  55                  60
Lys Thr Asp Asp Ala Lys Ile Tyr Lys Asn Thr Glu Ile Gln Ala Leu
65                  70                  75                  80
Ile Thr Ala Leu Gly Leu Gly Ile Lys Gly Glu Glu Phe Asp Ala Ser
                85                  90                  95
Gln Leu Arg Tyr His Arg Ile Val Ile Met Thr Asp Ala Asp Val Asp
            100                 105                 110
Gly Ala His Ile Arg Thr Leu Leu Leu Thr Phe Phe Tyr Arg Tyr Gln
        115                 120                 125
Arg Ser Leu Leu Glu Gln Gly Tyr Met Tyr Ile Ala Cys Pro Pro Leu
    130                 135                 140
Tyr Lys Leu Glu Arg Gly Arg Asn His Tyr Tyr Cys Tyr Asn Glu Arg
145                 150                 155                 160
Glu Leu Gln Glu Arg Ile Ala Thr Phe Pro Glu Asn Ala Asn Tyr Thr
                165                 170                 175
Ile
SEQ ID NO: 21
   SEQUENCE LENGTH: 660
   SEQUENCE TYPE: nucleic acid
   STRANDEDNESS: double
   TOPOLOGY: linear
   MOLECULE TYPE: genomic DNA
   ORIGINAL SOURCE
   ORGANISM: *Caulobacter crescentus*
   STRAIN: ATCC 15252
   SEQUENCE DESCRIPTION 
CGG GAT GGC GGC ACG CAC CTG TCG GCC TTT CGC GCG GCC CTG ACC CGG 48
Arg Asp Gly Gly Thr His Leu Ser Ala Phe Arg Ala Ala Leu Thr Arg
1               5                   10                  15
ATC ATC ACC AGC TAC GCC GAG AGC TCC GGC ATC CTG AAG AAG GAA AAG 96
Ile Ile Thr Ser Tyr Ala Glu Ser Ser Gly Ile Leu Lys Lys Glu Lys
            20                  25                  30
GTC AGC CTG GGC GGC GAA GAC AGC CGC GAG GGC CTG ACC TGC GTG CTG 144
Val Ser Leu Gly Gly Glu Asp Ser Arg Glu Gly Leu Thr Cys Val Leu
        35                  40                  45
TCG GTC AAG GTC CCG GAT CCG AAG TTC AGC TCG CAG ACC AAG GAC AAG 192
Ser Val Lys Val Pro Asp Pro Lys Phe Ser Ser Gln Thr Lys Asp Lys
    50                  55                  60
CTG GTC TCG TCC GAA GTG CGC CCC GCC GTT GAG GGC CTG GTG TCG GAA 240
Leu Val Ser Ser Glu Val Arg Pro Ala Val Glu Gly Leu Val Ser Glu
65                  70                  75                  80
GGT CTC TCG ACC TGG TTC GAG GAA CAT CCG AAC GAG GCC AAG GCG ATC 288
Gly Leu Ser Thr Trp Phe Glu Glu His Pro Asn Glu Ala Lys Ala Ile
                85                  90                  95
GTG ACC AAG ATC GCC GAG GCC GCC GCC GCC CGC GAG GCC GCC CGC AAG 336
 
Val Thr Lys Ile Ala Glu Ala Ala Ala Ala Arg Glu Ala Ala Arg Lys
            100                 105                 110
GCG CGA GAG CTG ACC CGC CGC AAG AGC GCG CTC GAC ATC ACC AGC CTG 384
Ala Arg Glu Leu Thr Arg Arg Lys Ser Ala Leu Asp Ile Thr Ser Leu
        115                 120                 125
CCC GGC AAG CTC GCC GAC TGC TCG GAA CGC GAT CCG GCC AAG TCC GAG 432
Pro Gly Lys Leu Ala Asp Cys Ser Glu Arg Asp Pro Ala Lys Ser Glu
    130                 135                 140
ATC TTC ATC GTC GAG GGC GAC TCG GCG GGC GGC TCG GCC AAG CAG GCC 480
Ile Phe Ile Val Glu Gly Asp Ser Ala Gly Gly Ser Ala Lys Gln Ala
145                 150                 155                 160
CGC AAC CGC GAC AAC CAG GCC GTT CTG CCC CTG CGC GGC AAG ATC CTG 528
Arg Asn Arg Asp Asn Gln Ala Val Leu Pro Leu Arg Gly Lys Ile Leu
                165                 170                 175
AAC GTC GAG CGG GCC CGC TTC GAC AAG ATG CTG TCG TCC GAC CAG ATC 576
Asn Val Glu Arg Ala Arg Phe Asp Lys Met Leu Ser Ser Asp Gln Ile
            180                 185                 190
GGC ACG CTG ATC ACC GCC CTG GGC GCG GGG ATC GGC CGC GAC GAC TTC 624
Gly Thr Leu Ile Thr Ala Leu Gly Ala Gly Ile Gly Arg Asp Asp Phe
        195                 200                 205
AAC CCG GAC AAG GTG CGC TAC CAC AAG ATC GTG CTG 660
Asn Pro Asp Lys Val Arg Tyr His Lys Ile Val Leu
    210                 215                 220
SEQ ID NO: 22
   SEQUENCE LENGTH: 220
   SEQUENCE TYPE: amino acid
   TOPOLOGY: unknown
   MOLECULE TYPE: protein
   ORIGINAL SOURCE
   ORGANISM: *Caulobacter crescentus*
   STRAIN: ATCC 15252
   SEQUENCE DESCRIPTION 
Arg Asp Gly Gly Thr His Leu Ser Ala Phe Arg Ala Ala Leu Thr Arg
1               5                   10                  15
Ile Ile Thr Ser Tyr Ala Glu Ser Ser Gly Ile Leu Lys Lys Glu Lys
            20                  25                  30
Val Ser Leu Gly Gly Glu Asp Ser Arg Glu Gly Leu Thr Cys Val Leu
        35                  40                  45
Ser Val Lys Val Pro Asp Pro Lys Phe Ser Ser Gln Thr Lys Asp Lys
    50                  55                  60
Leu Val Ser Ser Glu Val Arg Pro Ala Val Glu Gly Leu Val Ser Glu
65                  70                  75                  80
Gly Leu Ser Thr Trp Phe Glu Glu His Pro Asn Glu Ala Lys Ala Ile
                85                  90                  95
Val Thr Lys Ile Ala Glu Ala Ala Ala Ala Arg Glu Ala Ala Arg Lys
            100                 105                 110
Ala Arg Glu Leu Thr Arg Arg Lys Ser Ala Leu Asp Ile Thr Ser Leu
        115                 120                 125
Pro Gly Lys Leu Ala Asp Cys Ser Glu Arg Asp Pro Ala Lys Ser Glu
    130                 135                 140
Ile Phe Ile Val Glu Gly Asp Ser Ala Gly Gly Ser Ala Lys Gln Ala
145                 150                 155                 160
Arg Asn Arg Asp Asn Gln Ala Val Leu Pro Leu Arg Gly Lys Ile Leu
                165                 170                 175
Asn Val Glu Arg Ala Arg Phe Asp Lys Met Leu Ser Ser Asp Gln Ile
            180                 185                 190
Gly Thr Leu Ile Thr Ala Leu Gly Ala Gly Ile Gly Arg Asp Asp Phe
 
        195                 200                 205
Asn Pro Asp Lys Val Arg Tyr His Lys Ile Val Leu
    210                 215                 220
SEQ ID NO: 23
   SEQUENCE LENGTH: 1422
   SEQUENCE TYPE: nucleic acid
   STRANDEDNESS: double
   TOPOLOGY: linear
   MOLECULE TYPE: genomic DNA
   ORIGINAL SOURCE
   ORGANISM: *Cytophaga lytica*
   STRAIN: MBIC 1544
   SEQUENCE DESCRIPTION 
GAT AAA GAT TCA TAC AAA GTA TCT GGT GGT TTA CAC GGT GTA GGT GTA 48
Asp Lys Asp Ser Tyr Lys Val Ser Gly Gly Leu His Gly Val Gly Val
1               5                   10                  15
TCT TGT GTA AAC GCA TTA TCT AAT AAT TTA AAA GCT ACT GTT TAC AGA 96
Ser Cys Val Asn Ala Leu Ser Asn Asn Leu Lys Ala Thr Val Tyr Arg
            20                  25                  30
GAA GGT AAA ATA TGG GAG CAA GAG TAT GAA AGA GGT AAG GCT TTA TAT 144
Glu Gly Lys Ile Trp Glu Gln Glu Tyr Glu Arg Gly Lys Ala Leu Tyr
        35                  40                  45
CCG GTA AAA AGT ATT GGA GAA ACA GAG GAA ACA GGT ACT ATA GTT ACT 192
Pro Val Lys Ser Ile Gly Glu Thr Glu Glu Thr Gly Thr Ile Val Thr
    50                  55                  60
TTT TAC CCA GAT GAT ACT ATA TTT ACA CAA ACT ACA GAG TAT AAT TAT 240
Phe Tyr Pro Asp Asp Thr Ile Phe Thr Gln Thr Thr Glu Tyr Asn Tyr
65                  70                  75                  80
 
GAA ACG CTT TCT AAC AGA ATG CGA GAG TTG GCT TAC CTT AAT AAG GGA 288
Glu Thr Leu Ser Asn Arg Met Arg Glu Leu Ala Tyr Leu Asn Lys Gly
                85                  90                  95
GTT ACA ATT AGC ATT ACA GAT AAG AGA GTT AAA GAT GAA AAG GGA GAG 336
Val Thr Ile Ser Ile Thr Asp Lys Arg Val Lys Asp Glu Lys Gly Glu
            100                 105                 110
TTT TTA TCT GAA GTT TTT TAC TCT GAA GAA GGA CTA AAA GAA TTT ATT 384
Phe Leu Ser Glu Val Phe Tyr Ser Glu Glu Gly Leu Lys Glu Phe Ile
        115                 120                 125
AAG TTT TTA GAC GGT AAC AGA GAA CAA CTA ATA CGT GAT GTT GTT TCA 432
Lys Phe Leu Asp Gly Asn Arg Glu Gln Leu Ile Arg Asp Val Val Ser
    130                 135                 140
ATG GAA GGT GAA AAA AAC GGA ATT CCT GTT GAG GTT GCA ATG GTG TAC 480
Met Glu Gly Glu Lys Asn Gly Ile Pro Val Glu Val Ala Met Val Tyr
145                 150                 155                 160
AAT ACA TCA TAT TCA GAA AAT CTT CAC TCT TAC GTA AAT AAT ATT AAT 528
Asn Thr Ser Tyr Ser Glu Asn Leu His Ser Tyr Val Asn Asn Ile Asn
                165                 170                 175
ACA CAT GAA GGT GGT ACA CAC CTT TCA GGT TTT AGA AGA GGT TTA ACA 576
Thr His Glu Gly Gly Thr His Leu Ser Gly Phe Arg Arg Gly Leu Thr
            180                 185                 190
TCA ACC TTA AAA AAG TAT GCA GAT GCA TCT GGA ATG TTA GAC AAA TTA 624
Ser Thr Leu Lys Lys Tyr Ala Asp Ala Ser Gly Met Leu Asp Lys Leu
        195                 200                 205
AAG TTT GAG ATT CAG GGA GAT GAT TTT AGA GAA GGT TTA ACG GCT ATT 672
Lys Phe Glu Ile Gln Gly Asp Asp Phe Arg Glu Gly Leu Thr Ala Ile
    210                 215                 220
GTG TCT GTT AAA GTT GCA GAA CCT CAG TTT GAA GGG CAA ACA AAA ACT 720
Val Ser Val Lys Val Ala Glu Pro Gln Phe Glu Gly Gln Thr Lys Thr
 
225                 230                 235                 240

AAA TTA GGT AAC AGA GAA GTT TCT TCT GCA GTG AGC CAA GCT GTA TCA 768

Lys Leu Gly Asn Arg Glu Val Ser Ser Ala Val Ser Gln Ala Val Ser

                245                 250                 255

GAA ATG CTT ACC AAC TAT TTA GAA GAA AAC CCA GAT GAT GCT AAG GTA 816

Glu Met Leu Thr Asn Tyr Leu Glu Glu Asn Pro Asp Asp Ala Lys Val

            260                 265                 270

ATT GTA CAA AAA GTC ATT TTG GCA GCG CAA GCA CGT CA[?] GCG GCT ACA 864

Ile Val Gln Lys Val Ile Leu Ala Ala Gln Ala Arg His Ala Ala Thr

        275                 280                 285

AAA GCC CGT GAA ATG GTA CAG CGT AAA ACG GTA ATG AGT ATA GGT GGT 912

Lys Ala Arg Glu Met Val Gln Arg Lys Thr Val Met Ser Ile Gly Gly

    290                 295                 300

TTA CCA GGG AAA TTA TCA GAC TGT TCT GAG CAA GAT GCT ACA AAA TGC 960

Leu Pro Gly Lys Leu Ser Asp Cys Ser Glu Gln Asp Ala Thr Lys Cys

305                 310                 315                 320

GAA GTA TTC CTT GTA GAG GGA GAT TCG GCG GGT GGT ACT GCT AAA CAA 1008

Glu Val Phe Leu Val Glu Gly Asp Ser Ala Gly Gly Thr Ala Lys Gln

                325                 330                 335

GGT AGG GAC AGA AAC TTT CAG GCA ATA TTA CCG CTT CGT GGT AAA ATC 1056

Gly Arg Asp Arg Asn Phe Gln Ala Ile Leu Pro Leu Arg Gly Lys Ile

            340                 345                 350

TTA AAT GTT GAA AAA GCA ATG CAA CAT AAG GTT TTT GAA AAC GAA GAA 1104

Leu Asn Val Glu Lys Ala Met Gln His Lys Val Phe Glu Asn Glu Glu

        355                 360                 365

ATA AAA AAT ATT TAC ACA GCT TTA GGT GTT ACT ATT GGT ACA GAA GAA 1152

Ile Lys Asn Ile Tyr Thr Ala Leu Gly Val Thr Ile Gly Thr Glu Glu

    370                 375                 380

GAT AGT AAA GCC TTA AAC TTA GAA AAA TTA AGA TAC CAT AAA GTA GTT 1200
 
Asp Ser Lys Ala Leu Asn Leu Glu Lys Leu Arg Tyr His Lys Val Val
385                 390                 395                 400
ATT ATG TGT GAT GCC GAT GTA GAT GGT AGC CAC ATT GAA ACT TTA ATC 1248
Ile Met Cys Asp Ala Asp Val Asp Gly Ser His Ile Glu Thr Leu Ile
                405                 410                 415
CTT ACA TTC TTC TTC CGT TTT ATG AGG GAG TTA ATA GAA GGC GGT CAC 1296
Leu Thr Phe Phe Phe Arg Phe Met Arg Glu Leu Ile Glu Gly Gly His
            420                 425                 430
GTT TAT ATA GCA ACC CCA CCT TTA TAC TTG GTA AAA AAG GGA ACA AAA 1344
Val Tyr Ile Ala Thr Pro Pro Leu Tyr Leu Val Lys Lys Gly Thr Lys
        435                 440                 445
AAA CGT TAT GCT TGG AAT GAT AAA GAA CGA GAT GAG ATA GCA GAA AGC 1392
Lys Arg Tyr Ala Trp Asn Asp Lys Glu Arg Asp Glu Ile Ala Glu Ser
    450                 455                 460
TTT AAT GGT AGT GTT GGT ATA CAA AGA TAT 1422
Phe Asn Gly Ser Val Gly Ile Gln Arg Tyr
465                 470
SEQ ID NO: 24
   SEQUENCE LENGTH: 474
   SEQUENCE TYPE: amino acid
   TOPOLOGY: unknown
   MOLECULE TYPE: protein
   ORIGINAL SOURCE
   ORGANISM: *Cytophaga lytica*
   STRAIN: MBIC 1544
   SEQUENCE DESCRIPTION 
Asp Lys Asp Ser Tyr Lys Val Ser Gly Gly Leu His Gly Val Gly Val
1               5                   10                  15
 
Ser Cys Val Asn Ala Leu Ser Asn Asn Leu Lys Ala Thr Val Tyr Arg
            20                  25                  30
Glu Gly Lys Ile Trp Glu Gln Glu Tyr Glu Arg Gly Lys Ala Leu Tyr
        35                  40                  45
Pro Val Lys Ser Ile Gly Glu Thr Glu Glu Thr Gly Thr Ile Val Thr
    50                  55                  60
Phe Tyr Pro Asp Asp Thr Ile Phe Thr Gln Thr Thr Glu Tyr Asn Tyr
65                  70                  75                  80
Glu Thr Leu Ser Asn Arg Met Arg Glu Leu Ala Tyr Leu Asn Lys Gly
                85                  90                  95
Val Thr Ile Ser Ile Thr Asp Lys Arg Val Lys Asp Glu Lys Gly Glu
            100                 105                 110
Phe Leu Ser Glu Val Phe Tyr Ser Glu Glu Gly Leu Lys Glu Phe Ile
        115                 120                 125
Lys Phe Leu Asp Gly Asn Arg Glu Gln Leu Ile Arg Asp Val Val Ser
    130                 135                 140
Met Glu Gly Glu Lys Asn Gly Ile Pro Val Glu Val Ala Met Val Tyr
145                 150                 155                 160
Asn Thr Ser Tyr Ser Glu Asn Leu His Ser Tyr Val Asn Asn Ile Asn
                165                 170                 175
Thr His Glu Gly Gly Thr His Leu Ser Gly Phe Arg Arg Gly Leu Thr
            180                 185                 190
Ser Thr Leu Lys Lys Tyr Ala Asp Ala Ser Gly Met Leu Asp Lys Leu
        195                 200                 205
Lys Phe Glu Ile Gln Gly Asp Asp Phe Arg Glu Gly Leu Thr Ala Ile
    210                 215                 220
Val Ser Val Lys Val Ala Glu Pro Gln Phe Glu Gly Gln Thr Lys Thr
225                 230                 235                 240
Lys Leu Gly Asn Arg Glu Val Ser Ser Ala Val Ser Gln Ala Val Ser
 
                245                 250                 255
Glu Met Leu Thr Asn Tyr Leu Glu Glu Asn Pro Asp Asp Ala Lys Val
            260                 265                 270
Ile Val Gln Lys Val Ile Leu Ala Ala Gln Ala Arg His Ala Ala Thr
        275                 280                 285
Lys Ala Arg Glu Met Val Gln Arg Lys Thr Val Met Ser Ile Gly Gly
    290                 295                 300
Leu Pro Gly Lys Leu Ser Asp Cys Ser Glu Gln Asp Ala Thr Lys Cys
305                 310                 315                 320
Glu Val Phe Leu Val Glu Gly Asp Ser Ala Gly Gly Thr Ala Lys Gln
                325                 330                 335
Gly Arg Asp Arg Asn Phe Gln Ala Ile Leu Pro Leu Arg Gly Lys Ile
            340                 345                 350
Leu Asn Val Glu Lys Ala Met Gln His Lys Val Phe Glu Asn Glu Glu
        355                 360                 365
Ile Lys Asn Ile Tyr Thr Ala Leu Gly Val Thr Ile Gly Thr Glu Glu
    370                 375                 380
Asp Ser Lys Ala Leu Asn Leu Glu Lys Leu Arg Tyr His Lys Val Val
385                 390                 395                 400
Ile Met Cys Asp Ala Asp Val Asp Gly Ser His Ile Glu Thr Leu Ile
                405                 410                 415
Leu Thr Phe Phe Phe Arg Phe Met Arg Glu Leu Ile Glu Gly Gly His
            420                 425                 430
Val Tyr Ile Ala Thr Pro Pro Leu Tyr Leu Val Lys Lys Gly Thr Lys
        435                 440                 445
Lys Arg Tyr Ala Trp Asn Asp Lys Glu Arg Asp Glu Ile Ala Glu Ser
    450                 455                 460
Phe Asn Gly Ser Val Gly Ile Gln Arg Tyr
465                 470
SEQ ID NO: 25
   SEQUENCE LENGTH: 38
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION TGTAAAACGA CGGCCAGTCA YGCNGGNGGN AARTTYGA 38
SEQ ID NO: 26
   SEQUENCE LENGTH: 7
   SEQUENCE TYPE: amino acid
   TOPOLOGY: linear
   MOLECULE TYPE: peptide
   SEQUENCE DESCRIPTION 
His Ala Gly Gly Lys Phe Asp
1               5
SEQ ID NO: 27
   SEQUENCE LENGTH: 36
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION CTGCGTTCGT ATATGAGCNC CRTCNACRTC NGCRTC 36
SEQ ID NO: 28
   SEQUENCE LENGTH: 12
   SEQUENCE TYPE: amino acid
   TOPOLOGY: linear
   MOLECULE TYPE: peptide
   SEQUENCE DESCRIPTION 
Asp Ala Asp Val Asp Gly Ala His Ile Arg Thr Leu
1               5                   10
SEQ ID NO: 29
   SEQUENCE LENGTH: 41
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION GAAGTCATCA TGACCGTTCT GCAYGSNGGN GGNAARTTYG G 41
SEQ ID NO: 30
   SEQUENCE LENGTH: 14
   SEQUENCE TYPE: amino acid
   TOPOLOGY: linear
   MOLECULE TYPE: peptide
   SEQUENCE DESCRIPTION 
Glu Val Leu Met Thr Val Leu His Ala Gly Gly Lys Phe Gly
1               5                   10
SEQ ID NO: 31
   SEQUENCE LENGTH: 44
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION AGCAGGGTAC GGATGTGCGA GCCRTCNACR TCNGCRTCNG TGAT 44
SEQ ID NO: 32
   SEQUENCE LENGTH: 15
   SEQUENCE TYPE: amino acid
   TOPOLOGY: linear
   MOLECULE TYPE: peptide
   SEQUENCE DESCRIPTION 
Met Thr Asp Ala Asp Val Asp Gly Ser His Ile Arg Thr Leu Leu
1               5                   10                  15
SEQ ID NO: 33
   SEQUENCE LENGTH: 32
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION CAGGAAACAG CTATGACCAR RTGNGTNCCN CC 32
SEQ ID NO: 34
   SEQUENCE LENGTH: 5
   SEQUENCE TYPE: amino acid
   TOPOLOGY: linear
   MOLECULE TYPE: peptide
   SEQUENCE DESCRIPTION 
Gly Gly Thr His Leu
1               5
SEQ ID NO: 35
   SEQUENCE LENGTH: 34
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION GCAACGAGAT CAACACTCMN GARGGNGGNA CNCA 34
SEQ ID NO: 36
   SEQUENCE LENGTH: 11
   SEQUENCE TYPE: amino acid
   TOPOLOGY: linear
   MOLECULE TYPE: peptide
   SEQUENCE DESCRIPTION 
Asn Asn Ile Asn Thr His Glu Gly Gly Thr His
1               5                   10
SEQ ID NO: 37
   SEQUENCE LENGTH: 11
   SEQUENCE TYPE: amino acid
   TOPOLOGY: linear
   MOLECULE TYPE: peptide
   SEQUENCE DESCRIPTION Asn Asn Ile Asn Thr Pro Glu Gly Gly Thr His 1                    5                    10
SEQ ID NO: 38
   SEQUENCE LENGTH: 35
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION TGTAAAACGA CGGCCAGTAR YTTNKYYTTN GTYTG 35
SEQ ID NO: 39
   SEQUENCE LENGTH: 6
   SEQUENCE TYPE: amino acid
   TOPOLOGY: linear
   MOLECULE TYPE: peptide
   SEQUENCE DESCRIPTION 
Gln Thr Lys Thr Lys Leu
1               5
SEQ ID NO: 40
   SEQUENCE LENGTH: 6
   SEQUENCE TYPE: amino acid
   TOPOLOGY: linear
   MOLECULE TYPE: peptide
   SEQUENCE DESCRIPTION 
Gln Thr Lys Asp Lys Leu
1               5
SEQ ID NO: 41
   SEQUENCE LENGTH: 35
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION TAGGCTAGCT GACCGTAAGA YGCNGAYRTN GAYGG 35
SEQ ID NO: 42
   SEQUENCE LENGTH: 6
   SEQUENCE TYPE: amino acid
   TOPOLOGY: linear
   MOLECULE TYPE: peptide
   SEQUENCE DESCRIPTION 
Asp Ala Asp Val Asp Gly
1               5
SEQ ID NO: 43
   SEQUENCE LENGTH: 36
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION CCATAGCTGC GTAGCATTCA TYTCNCCNAR NCCYTT 36
SEQ ID NO: 44
   SEQUENCE LENGTH: 12
   SEQUENCE TYPE: amino acid
   TOPOLOGY: linear
   MOLECULE TYPE: peptide
   SEQUENCE DESCRIPTION 
Lys Gly Leu Gly Glu Met Asn Ala Thr Gln Leu Trp
1               5                   10
SEQ ID NO: 45
   SEQUENCE LENGTH: 41
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION CAGGAAACAG CTATGACCAA RMGNCCNGSN ATGTAYATHG G 41
SEQ ID NO: 46
   SEQUENCE LENGTH: 8
   SEQUENCE TYPE: amino acid
   TOPOLOGY: linear
   MOLECULE TYPE: peptide
   SEQUENCE DESCRIPTION 
Lys Arg Pro Ala Met Tyr Ile Gly
1               5
SEQ ID NO: 47
   SEQUENCE LENGTH: 8
   SEQUENCE TYPE: amino acid
   TOPOLOGY: linear
   MOLECULE TYPE: peptide
   SEQUENCE DESCRIPTION 
Lys Arg Pro Gly Met Tyr Ile Gly
1               5
SEQ ID NO: 48
   SEQUENCE LENGTH: 38
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION TGTAAAACGA CGGCCAGTCC NCCNGCNSWR TCNCCYTC 38
SEQ ID NO: 49
   SEQUENCE LENGTH: 7
   SEQUENCE TYPE: amino acid
   TOPOLOGY: linear
   MOLECULE TYPE: peptide
   SEQUENCE DESCRIPTION 
Glu Gly Asp Ser Ala Gly Gly
1               5
SEQ ID NO: 50
   SEQUENCE LENGTH: 39
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION TGTAAAACGA CGGCCAGTCA TNGTNGTNTC CCANARYTG 39
SEQ ID NO: 51
   SEQUENCE LENGTH: 7
   SEQUENCE TYPE: amino acid
   TOPOLOGY: linear
   MOLECULE TYPE: peptide
   SEQUENCE DESCRIPTION 
Gln Leu Trp Glu Thr Thr Met
1               5
SEQ ID NO: 52
   SEQUENCE LENGTH: 7
   SEQUENCE TYPE: amino acid
   TOPOLOGY: linear
   MOLECULE TYPE: peptide
   SEQUENCE DESCRIPTION 
Gln Leu Trp Asp Thr Thr Met
1               5
SEQ ID NO: 53
   SEQUENCE LENGTH: 41
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION GAAGTCATCA TGACCGTTCT GCAYGCNGGN GGNAARTTYG A 41
SEQ ID NO: 54
   SEQUENCE LENGTH: 14
   SEQUENCE TYPE: amino acid
   TOPOLOGY: linear
   MOLECULE TYPE: peptide
   SEQUENCE DESCRIPTION 
Glu Val Ile Met Thr Val Leu His Ala Gly Gly Lys Phe Asp
1               5                   10
SEQ ID NO: 55
   SEQUENCE LENGTH: 14
   SEQUENCE TYPE: amino acid
   TOPOLOGY: linear
   MOLECULE TYPE: peptide
   SEQUENCE DESCRIPTION 
Glu Val Ile Met Thr Val Leu His Ala Gly Gly Lys Phe Asn
1               5                   10
SEQ ID NO: 56
   SEQUENCE LENGTH: 14
   SEQUENCE TYPE: amino acid
   TOPOLOGY: linear
   MOLECULE TYPE: peptide
   SEQUENCE DESCRIPTION 
Glu Val Ile Met Thr Val Leu His Ala Gly Gly Lys Phe Glu
1               5                   10
SEQ ID NO: 57
   SEQUENCE LENGTH: 14
   SEQUENCE TYPE: amino acid
   TOPOLOGY: linear
   MOLECULE TYPE: peptide
   SEQUENCE DESCRIPTION 
Glu Val Ile Met Thr Val Leu His Ala Gly Gly Lys Phe Lys
1               5                   10
SEQ ID NO: 58
   SEQUENCE LENGTH: 38
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION TGTAAAACGA CGGCCAGTGC NGGRTCYTTY TCYTGRCA 38
SEQ ID NO: 59
   SEQUENCE LENGTH: 7
   SEQUENCE TYPE: amino acid
   TOPOLOGY: linear
   MOLECULE TYPE: peptide
   SEQUENCE DESCRIPTION 
Cys Gln Glu Lys Asp Pro Ala
1               5
SEQ ID NO: 60
   SEQUENCE LENGTH: 40
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION GAAGTCATCA TGACCGTTCT GCAACNAAYA AYATHCCNCA 40
SEQ ID NO: 61
   SEQUENCE LENGTH: 6
   SEQUENCE TYPE: amino acid
   TOPOLOGY: linear
   MOLECULE TYPE: peptide
   SEQUENCE DESCRIPTION 
Thr Asn Asn Ile Pro Gln
1               5
SEQ ID NO: 62
   SEQUENCE LENGTH: 38
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION TGTAAAACGA CGGCCAGTAA YTTNGGNTCN GGNACYTT 38
SEQ ID NO: 63
   SEQUENCE LENGTH: 7
   SEQUENCE TYPE: amino acid
   TOPOLOGY: linear
   MOLECULE TYPE: peptide
   SEQUENCE DESCRIPTION 
Lys Val Pro Asp Pro Lys Phe
1               5
SEQ ID NO: 64
   SEQUENCE LENGTH: 7
   SEQUENCE TYPE: amino acid
   TOPOLOGY: linear
   MOLECULE TYPE: peptide
   SEQUENCE DESCRIPTION 
Lys Val Pro Glu Pro Lys Phe
1               5
SEQ ID NO: 65
   SEQUENCE LENGTH: 35
   SEQUENCE TYPE: nucleic acid
   STRANDEDNESS: single
   TOPOLOGY: linear
   MOLECULE TYPE: other nucleic acid synthetic DNA
   SEQUENCE DESCRIPTION CAGGAAACAG CTATGACCGC NMRNMRNGCN MGNGA 35
SEQ ID NO: 66
   SEQUENCE LENGTH: 6
   SEQUENCE TYPE: amino acid
   TOPOLOGY: linear
   MOLECULE TYPE: peptide
   SEQUENCE DESCRIPTION Ala Arg Arg Ala Arg Glu 1                    5
SEQ ID NO: 67
   SEQUENCE LENGTH: 6
   SEQUENCE TYPE: amino acid
   TOPOLOGY: linear
   MOLECULE TYPE: peptide
   SEQUENCE DESCRIPTION 
Ala Arg Lys Ala Arg Glu
1               5
SEQ ID NO: 68
   SEQUENCE LENGTH: 6
   SEQUENCE TYPE: amino acid TOPOLOGY: linear
   MOLECULE TYPE: peptide
   SEQUENCE DESCRIPTION

Ala Lys Lys Ala Arg Glu
1               5

## Claims

1. A set of primers suitable for the identification of a microorganism, which primer set comprises two primers comprising the nucleotide sequences of:
(i) SEQ ID NO: 33 and SEQ ID NO: 27;
(ii) SEQ ID NO: 25 and SEQ ID NO: 35;
(iii) SEQ ID NO: 29 and SEQ ID NO: 38;
(iv) SEQ ID NO: 41 and SEQ ID NO: 43;
(v) SEQ ID NO: 45 and SEQ ID NO: 48;
(vi) SEQ ID NO: 53 and SEQ ID NO: 62;
(vii) SEQ ID NO: 53 and SEQ ID NO: 58;
(viii) SEQ ID NO: 65 and SEQ ID NO: 50;
(ix) SEQ ID NO: 60 and SEQ ID NO: 31; or
(x) SEQ ID NO: 25 and SEQ ID NO: 43.

2. A method for detecting a microorganism, which method comprises:
(i) amplifying DNA from a sample by PCR using a set of primers according to claim 1; and
(ii) identifying whether the sample comprises a microorganism based on the nucleotide sequence of any amplified DNA fragment.

3. A method for identifying a microorganism, which method comprises:
(i) amplifying DNA from the microorganism by PCR using a set of primers according to claim 1; and
(ii) identifying the microorganism based on the nucleotide sequence of the amplified DNA fragment.

4. A method for determining the sequence of a gyrB gene of a microorganism, which method comprises:
(i) amplifying DNA from the microorganism by PCR using a set of primers according to claim 1; and
(ii) determining the nucleotide sequence of the gyrB gene of the microorganism based on the sequence of the amplified DNA fragment.

## Patentansprüche

1. Satz von Primern, geeignet für die Identifizierung eines Mikroorganismus, wobei der Satz von Primern zwei Primer umfasst, umfassend die Nucleotidsequenz von:
(i) SEQ ID NO: 33 und SEQ ID NO: 27;
(ii) SEQ ID NO: 25 und SEQ ID NO: 35;
(iii) SEQ ID NO: 29 und SEQ ID NO: 38;
(iv) SEQ ID NO: 41 und SEQ ID NO: 43;
(v) SEQ ID NO: 45 und SEQ ID NO: 48;
(vi) SEQ ID NO: 53 und SEQ ID NO: 62;
(vii) SEQ ID NO: 53 und SEQ ID NO: 58;
(viii) SEQ ID NO: 65 und SEQ ID NO: 50;
(ix) SEQ ID NO: 60 und SEQ ID NO: 31; oder
(x) SEQ ID NO: 25 und SEQ ID NO: 43.

2. Verfahren zum Nachweis eines Mikroorganismus, wobei das Verfahren umfasst:
(i) Amplifizieren von DNA aus einer Probe durch PCR unter Verwendung eines Satzes von Primern nach Anspruch 1; und
(ii) Identifizieren, ob die Probe einen Mikroorganismus umfasst, auf der Grundlage der Nucleotidsequenz eines amplifizierten DNA-Fragments.

3. Verfahren zur Identifizierung eines Mikroorganismus, wobei das Verfahren umfasst:
(i) Amplifizieren von DNA des Mikroorganismus durch PCR unter Verwendung eines Satzes von Primern nach Anspruch 1; und
(ii) Identifizieren des Mikroorganismus auf der Grundlage der Nucleotidsequenz des amplifizierten DNA-Fragments.

4. Verfahren zur Bestimmung der Sequenz eines gyrB-Gens eines Mikroorganismus, wobei das Verfahren umfasst:
(i) Amplifizieren von DNA des Mikroorganismus durch PCR unter Verwendung eines Satzes von Primern nach Anspruch 1; und
(ii) Bestimmen der Nucleotidsequenz des gyrB-Gens des Mikroorganismus auf der Grundlage der Sequenz des amplifizierten DNA-Fragments.

## Revendications

1. Ensemble d'amorces convenant pour l'identification d'un microorganisme, lequel ensemble d'amorces comprend deux amorces comprenant les séquences nucléotidiques de :
(i) SEQ ID NO : 33 et SEQ ID NO : 27;
(ii) SEQ ID NO : 25 et SEQ ID NO : 35 ;
(iii) SEQ ID NO : 29 et SEQ ID NO : 38 ;
(iv) SEQ ID NO : 41 et SEQ ID NO : 43 :
(v) SEQ ID NO : 45 et SEQ ID NO : 48 ;
(vi) SEQ ID NO : 53 et SEQ ID NO : 62 ;
(vii) SEQ ID NO : 53 et SEQ ID NO : 58 ;
(viii) SEQ ID NO : 65 et SEQ ID NO : 50 ;
(ix) SEQ ID NO : 60 et SEQ ID NO : 31 ; ou
(x) SEQ ID NO : 25 et SEQ ID NO : 43.

2. Procédé pour détecter un microorganisme, lequel procédé comprend :
(i) l'amplification d'ADN d'un échantillon par PCR au moyen d'un ensemble d'amorces selon la revendication 1 ; et
(ii) l'identification du fait que l'échantillon comprend un microorganisme sur la base de la séquence nucléotidique de tout fragment d'ADN amplifié.

3. Procédé pour identifier un microorganisme, lequel procédé comprend :
(i) l'amplification d'ADN du microorganisme par PCR au moyen d'un ensemble d'amorces selon la revendication 1 ; et
(ii) l'identification du microorganisme sur la base de la séquence nucléotidique du fragment d'ADN amplifié.

4. Procédé pour déterminer la séquence d'un gène gyrB d'un microorganisme, lequel procédé comprend
(i) l'amplification d'ADN du microorganisme par PCR au moyen d'un ensemble d'amorces selon la revendication 1 ; et
(ii) la détermination de la séquence nucléotidique du gène gyrB du microorganisme sur la base de la séquence du fragment d'ADN amplifié.
